# EUROPEAN PATENT SPECIFICATION

(11) **EP 4 324 296 B1**
(45) Date of publication and mention of the grant of the patent: **20.11.2024**
(21) Application number: 22721400.4
(22) Date of filing: 11.04.2022
(51) Int. Cl.: H05B 45/20, H05B 47/105, F21V 14/00, C02F 1/32, A61L 2/10, A61L 9/20, A61L 2/24, A61L 2/26

(54) **LED ARRAY FOR DISINFECTION COMPRISING DIFFERENT TYPES OF LEDS**
LED-ANORDNUNG ZUR DESINFEKTION MIT VERSCHIEDENEN ARTEN VON LEDS
RÉSEAU DE DEL POUR LA DÉSINFECTION COMPRENANT DIFFÉRENTS TYPES DE DEL

(30) Priority: 15.04.2021 EP 21168637
(43) Date of publication of application: 21.02.2024
(73) Proprietor: Signify Holding B.V., 5656 AE Eindhoven (NL)
(72) Inventor: VAN BOMMEL, Ties, 5656 AE Eindhoven (NL)
(74) Representative: Verweij, Petronella Daniëlle
(86) International application number: PCT/EP2022/059562
(87) International publication number: WO 2022/218894

(56) References cited:
- CN-U- 211 551 554
- KR-A- 20210 004 549
- US-A1- 2019 022 263

## Description

### FIELD OF THE INVENTION

The invention relates to a radiation generating system. The invention also relates to a disinfection device, comprising the light generating system. Yet further, the invention relates to a method for treating a gas or a surface.

### BACKGROUND OF THE INVENTION

Sources of radiation are known in the art. US2006/02619291, for instance, describes an apparatus for selectively emitting one or more of a plurality of wavelength distributions of radiation, comprising: a primary UV radiation source, disposed in a housing, wherein said primary UV radiation source produces a primary wavelength distribution; at least one wavelength-transforming material, deposed external to the envelope of said primary UV radiation source, that in response to irradiation by said primary UV radiation source, emits a transformed radiation; a selection mechanism deposed internal to the housing, wherein said selection mechanism is substantially non-removably coupled to the housing; wherein said selection mechanism places at least one selected portion of said at least one wavelength-transforming materials relative to said primary UV radiation source; a reflector unit disposed proximate to said primary UV radiation source wherein said reflector unit directs the primary wavelength distribution radiation from said primary UV radiation source onto said at least one wavelength-transforming material; and wherein said reflector unit is also deposed relative to said primary UV radiation source such that non-selected portions of said at least one wavelength-transforming materials are shaded from irradiation by said primary UV radiation source. Said wavelength transforming filter is constructed to form an opening in at least a portion of said wavelength transforming filter thereby permitting said primary wavelength distribution to emit there-thru unattenuated.

US2019/022263A1 discloses a luminaire for disinfecting a target surface includes a disinfecting light source, a non-disinfecting light source, a beam angle adjustor, a motion sensor, and a distance sensor. The radiance of the disinfecting light is calculated based on detected distance to a target surface and beam angle, and may be selected to achieve a predetermined irradiance of the target surface. If no motion is detected by the motion sensor, then the disinfecting light source is set to ON and the non-disinfecting light source is set to OFF. If motion is detected and a beam intercept is not detected by the distance sensor, then the disinfecting light source is set to DIM and the non-disinfecting light source is set to ON. If motion is detected and a beam intercept is detected, then the disinfecting light source is set to OFF and the non-disinfecting light source is set to ON.

KR20210004549A discloses a sterilization unit capable of increasing a flow path and residence time of a fluid and, more specifically, to a sterilization unit comprising: a flow passage unit including a flow passage extending in a first direction; a fixing unit disposed in the flow passage unit and extending in a direction different from the extending direction of the flow passage; and a light source disposed on the fixing unit and emitting ultraviolet rays. The fluid supplied to the flow passage unit flows in the first direction, the fixing unit extends in a direction perpendicular to the first direction, and an optical axis of the light source is parallel to the first direction.

### SUMMARY OF THE INVENTION

UV light has been used for disinfection for over 100 years. Wavelengths between about 190 nm and 300 nm may be strongly absorbed by nucleic acids, which may result in defects in an organism's genome. This may be desired for inactivating (killing), bacteria and viruses, but may also have undesired side effects for humans. Therefore, the selection of wavelength of radiation, intensity of radiation and duration of irradiation may be limited in environments where people may reside such as offices, public transport, cinema's, restaurants, shops, etc., thus limiting the disinfection capacity. Especially in such environments, additional measures of disinfection may be advantageous to prevent the spread of bacteria and viruses such as influenza or novel (corona) viruses like COVID-19, SARS and MERS.

The ultraviolet wavelength range is defined as light in a wavelength range from 100 to 380 nm and can be divided into different types of UV light / UV wavelength ranges (Table 1). Different UV wavelengths of radiation may have different properties and thus may have different compatibility with human presence and may have different effects when used for disinfection (Table 1).

**Table 1: Properties of different types of UV wavelength light**

| Name | Short name | Wavelength (nm) | (Relative) sterilization effectiveness | | Safe Radiation | Vitamin D generation | Ozone generation |
|---|---|---|---|---|---|---|---|
| | | | Bacteria | Viruses | | | |
| Violet | V | 380-420 | +/- | - | + | | |
| Ultraviolet A | UV-A | 315-380 | + | - | + | | |
| Ultraviolet B | UV-B | 280-315 | + | +/- | +/- | + | |
| Near ultraviolet C | Near UV-C | 230-280 | + | + | - | | |
| Far ultraviolet | Far UV-C | 190-230 | + | + | + | | +/- |
| Extreme ultraviolet C | Extreme UV-C | 100-190 | + | + | - | | + |

Each UV type / wavelength range may have different benefits and/or drawbacks. Relevant aspects may be (relative) sterilization effectiveness, safety (regarding radiation), and ozone production (as result of its radiation). Depending on an application a specific type of UV light or a specific combination of UV light types may be selected and provides superior performance over other types of UV light. UV-A may be (relatively) safe and may inactivate (kill) bacteria, but may be less effective in inactivating (killing) viruses. UV-B may be (relatively) safe when a low dose (i.e. low exposure time and/or low intensity) is used, may inactivate (kill) bacteria, and may be moderately effective in inactivating (killing) viruses. UV-B may also have the additional benefit that it can be used effectively in the production of vitamin D in a skin of a person or animal. Near UV-C may be relatively unsafe, but may effectively inactivating, especially kill bacteria and viruses. Far UV-C may also be effective in inactivating (killing) bacteria and viruses, but may be (relatively to other UV-C wavelength ranges) (rather) safe. Far-UV light may generate some ozone which may be harmful for human beings and animals. Extreme UV-C may also be effective in inactivating (killing) bacteria and viruses, but may be relatively unsafe. Extreme UV-C may generate ozone which may be undesired when exposed to human beings or animals. In some application ozone may be desired and may contribute to disinfection, but then its shielding from humans and animals may be desired. Hence, in the table "+" for ozone production especially implies that ozone is produced which may be useful for disinfection applications, but may be harmful for humans / animals when they are exposed to it. Hence, in many applications this "+" may actually be undesired while in others, it may be desired. The terms "inactivating" and "killing" with respect to a virus may herein especially refer to damaging the virus in such a way that the virus can no longer infect and/or reproduce in a host cell, i.e., the virus may be (essentially) harmless after inactivation or killing.

Hence, in embodiments, the light may comprise a wavelength in the UV-A range. In further embodiments, the light may comprise a wavelength in the UV-B range. In further embodiments, the light may comprise a wavelength in the Near UV-C range. In further embodiments, the light may comprise a wavelength in the Far UV-C range. In further embodiments, the light may comprise a wavelength in the extreme UV-C range. The Near UV-C, the Far UV-C and the extreme UV-C ranges may herein also collectively be referred to as the UV-C range. Hence, in embodiments, the light may comprise a wavelength in the UV-C range. In other embodiments, the light may comprise violet radiation.

It appears desirable to provide systems, that provide radiation having specifically selected wavelengths at specifically selected locations only and/or having a spatially controllable power distribution in dependence of the wavelength. Hence, it is an aspect of the invention to provide an alternative radiation generating system which preferably further at least partly obviates one or more of above-described drawbacks. The present invention may have as object to overcome or ameliorate at least one of the disadvantages of the prior art, or to provide a useful alternative.

Therefore, in a first aspect, the invention provides a radiation generating system (which may also be indicated as "light generating system") comprising one or more first light sources (which may also be indicated as "first radiation sources" or "first sources of first radiation") and one or more second light sources (which may also be indicated as "second radiation sources" or "second sources of second radiation"). In embodiments, one or more of the one or more first light sources and the one or more second light sources may especially be solid state light sources. Especially, the one or more first light sources are configured to generate in an operational mode first light source light (or "first light source radiation") having a first centroid wavelength (λc1). In embodiments, the first centroid wavelength (λc1) may be defined within a wavelength range of at maximum 420 nm, such as at maximum 380 nm. Especially, the one or more second light sources are configured to generate in an operational mode second light source light ("second light source radiation") having a second centroid wavelength (λc2). In embodiments, the second centroid wavelength (λc2) may be defined within the wavelength range of at maximum 420 nm, such as at maximum 380 nm. In specific embodiments the second centroid wavelength (λc2) > the first centroid wavelength (λc1). In embodiments of the invention, the radiation generating system may be configured to provide a beam of radiation comprising one or more of (i) the first light source light and (ii) the second light source light. Especially, the first light source light has a first beam angle (α1), and the second light source light has a second beam angle (α2). In embodiments, the first beam angle (α1) < the second beam angle (α2). The first light source light may be provided as a (first) beam of first light source light and the second light source light may be provided as a (second) beam of second light source light. Hence, the radiation generating system may be configured to provide a beam of radiation comprising one or more of (i) the first beam comprising first light source light having the first beam angle (α1) defined by full width half maxima and (ii) the second beam comprising second light source light having the second beam angle (α2) defined by full width half maxima; wherein the first beam and the second beam may especially at least partly overlap. Therefore, in specific embodiments the invention provides a radiation generating system comprising one or more first light sources and one or more second light sources, wherein: (i) the one or more first light sources and the one or more second light sources are solid state light sources; (ii) the one or more first light sources are configured to generate in an operational mode first light source light having a first centroid wavelength (λc1) defined within a wavelength range of at maximum 380 nm; (iii) the one or more second light sources are configured to generate in an operational mode second light source light having a second centroid wavelength (λc2) defined within the wavelength range of at maximum 420 nm; wherein the second centroid wavelength (λc2) > the first centroid wavelength (λc1); (iv) the radiation generating system is configured to provide a beam of radiation comprising one or more of (i) the first light source light and (ii) the second light source light; wherein the first light source light has a first beam angle (α1), wherein the second light source light has a second beam angle (α2), and wherein the first beam angle (α1) < the second beam angle (α2). Especially, the beam angles are defined by full width half maxima. Hence, in embodiments the radiation generating system comprising one or more first light sources and one or more second light sources, wherein: (A) the one or more first light sources and the one or more second light sources are solid state light sources; (B) the one or more first light sources are configured to generate in an operational mode first light source light having a first centroid wavelength (λc1) defined within a wavelength range of at maximum 380 nm; (C) the one or more second light sources are configured to generate in an operational mode second light source light having a second centroid wavelength (λc2) defined within the wavelength range of at maximum 420 nm; wherein the second centroid wavelength (λc2) > the first centroid wavelength (λc1); and (D) the radiation generating system is configured to provide a beam of radiation comprising one or more of (i) a first beam comprising first light source light having a first beam angle (α1) defined by full width half maxima and (ii) a second beam comprising second light source light having s a second beam angle (α2) defined by full width half maxima; wherein the first beam and the second beam at least partly overlap, and wherein the first beam angle (α1) < the second beam angle (α2).

Such a system may provide specifically selected wavelengths at specifically selected locations only. Especially, the center of a beam of radiation may comprise radiation that is more efficient in inactivating bacteria and/or viruses. In this way, the radiation that may be harmful for humans may be directed at specific locations only and may be surrounded by less harmful radiation. Hence, with such system the spatial distribution of the spectral power may be controlled. For instance, more effective and/or more dangerous radiation may have a narrower beam width than less effective and/or less dangerous radiation.

As indicated above, the radiation generating system may comprise one or more first light sources and one or more second light sources. The one or more first light sources may in embodiments all be of the same type, or may in other embodiments be of two or more, such as two, different types. The one or more second light sources may in embodiments all be of the same type, or may in other embodiments be of two or more, such as two or three or four, such as two or three, different types.

The term "light source" may in principle relate to any light source known in the art. It may be a conventional (tungsten) light bulb, a low-pressure mercury lamp, a high pressure mercury lamp, a fluorescent lamp, a LED (light emissive diode). In a specific embodiment, the light source comprises a solid state LED light source (such as a LED or laser diode (or "diode laser")). The term "light source" may also relate to a plurality of light sources, such as 2-200 (solid state) LED light sources. Hence, the term LED may also refer to a plurality of LEDs. Further, the term "light source" may in embodiments also refer to a so-called chips-on-board (COB) light source. The term "COB" especially refers to LED chips in the form of a semiconductor chip that is neither encased nor connected but directly mounted onto a substrate, such as a PCB. Hence, a plurality of light semiconductor light source may be configured on the same substrate. In embodiments, a COB is a multi-LED chip configured together as a single lighting module.

The light source has a light escape surface. Referring to conventional light sources such as light bulbs or fluorescent lamps, it may be outer surface of the glass or quartz envelope. For LED's it may for instance be the LED die, or when a resin is applied to the LED die, the outer surface of the resin. In principle, it may also be the terminal end of a fiber. The term escape surface especially relates to that part of the light source, where the light actually leaves or escapes from the light source. The light source is configured to provide a beam of light. This beam of light (thus) escapes from the light exit surface of the light source.

The term "light source" may refer to a semiconductor light-emitting device, such as a light emitting diode (LEDs), a resonant cavity light emitting diode (RCLED), a vertical cavity laser diode (VCSELs), an edge emitting laser, etc... The term "light source" may also refer to an organic light-emitting diode, such as a passive-matrix (PMOLED) or an active-matrix (AMOLED). In a specific embodiment, the light source comprises a solid-state light source (such as a LED or laser diode). In an embodiment, the light source comprises a LED (light emitting diode). The terms "light source" or "solid state light source" may also refer to a superluminescent diode (SLED).

The term LED may also refer to a plurality of LEDs. Further, the term "light source" may in embodiments also refer to a so-called chips-on-board (COB) light source. The term "COB" especially refers to LED chips in the form of a semiconductor chip that is neither encased nor connected but directly mounted onto a substrate, such as a PCB. Hence, a plurality of semiconductor light sources may be configured on the same substrate. In embodiments, a COB is a multi-LED chip configured together as a single lighting module.

The term "light source" may also relate to a plurality of (essentially identical (or different)) light sources, such as 2-2000 solid state light sources. In embodiments, the light source may comprise one or more micro-optical elements (array of micro lenses) downstream of a single solid-state light source, such as a LED, or downstream of a plurality of solid-state light sources (i.e. e.g. shared by multiple LEDs). In embodiments, the light source may comprise a LED with on-chip optics. In embodiments, the light source comprises a pixelated single LEDs (with or without optics) (offering in embodiments on-chip beam steering).

In embodiments, the light source may be configured to provide primary radiation, which is used as such, such as e.g. a blue light source, like a blue LED, or a green light source, such as a green LED, and a red light source, such as a red LED. Such LEDs, which may not comprise a luminescent material ("phosphor") may be indicated as direct color LEDs. In other embodiments, however, the light source may be configured to provide primary radiation and part of the primary radiation is converted into secondary radiation. Secondary radiation may be based on conversion by a luminescent material. The secondary radiation may therefore also be indicated as luminescent material radiation. The luminescent material may in embodiments be comprised by the light source, such as a LED with a luminescent material layer or dome comprising luminescent material. Such LEDs may be indicated as phosphor converted LEDs or PC LEDs (phosphor converted LEDs). In other embodiments, the luminescent material may be configured at some distance ("remote") from the light source, such as a LED with a luminescent material layer not in physical contact with a die of the LED. Hence, in specific embodiments the light source may be a light source that during operation emits at least light at wavelength selected from the range of 380-470 nm. However, other wavelengths may also be possible. This light may partially be used by the luminescent material. In embodiments, the light generating device may comprise a luminescent material. In embodiments, the light generating device may comprise a PC LED. In other embodiments, the light generating device may comprise a direct LED (i.e. no phosphor). In embodiments, the light generating device may comprise a laser device, like a laser diode. In embodiments, the light generating device may comprise a superluminescent diode.

The term "laser light source" especially refers to a laser. Such laser may especially be configured to generate laser light source light having one or more wavelengths in the UV, visible, or infrared, especially having a wavelength selected from the spectral wavelength range of 200-2000 nm, such as 300-1500 nm. The term "laser" especially refers to a device that emits light through a process of optical amplification based on the stimulated emission of electromagnetic radiation. Especially, in embodiments the term "laser" may refer to a solid-state laser. In specific embodiments, the terms "laser" or "laser light source", or similar terms, refer to a laser diode (or diode laser).

Hence, in embodiments the light source comprises a laser light source. In embodiments, the terms "laser" or "solid state laser" may refer to one or more of cerium doped lithium strontium (or calcium) aluminum fluoride (Ce:LiSAF, Ce:LiCAF), chromium doped chrysoberyl (alexandrite) laser, chromium ZnSe (Cr:ZnSe) laser, divalent samarium doped calcium fluoride (Sm:CaF₂) laser, Er:YAG laser, erbium doped and erbium-ytterbium codoped glass lasers, F-Center laser, holmium YAG (Ho:YAG) laser, Nd:YAG laser, NdCrYAG laser, neodymium doped yttrium calcium oxoborate Nd:YCa₄O(BO₃)₃ or Nd:YCOB, neodymium doped yttrium orthovanadate (Nd:YVO₄) laser, neodymium glass (Nd:glass) laser, neodymium YLF (Nd:YLF) solid-state laser, promethium 147 doped phosphate glass (147Pm³⁺:glass) solid-state laser, ruby laser (AL₂O₃:Cr³⁺), thulium YAG (Tm:YAG) laser, titanium sapphire (Ti:sapphire; Al₂O₃:Ti³⁺) laser, trivalent uranium doped calcium fluoride (U:CaF₂) solid-state laser, Ytterbium doped glass laser (rod, plate/chip, and fiber), Ytterbium YAG (Yb:YAG) laser, Yb₂O₃ (glass or ceramics) laser, etc.

In embodiments, the terms "laser" or "solid state laser" may refer to one or more of a semiconductor laser diode, such as GaN, InGaN, AlGaInP, AlGaAs, InGaAsP, lead salt, vertical cavity surface emitting laser (VCSEL), quantum cascade laser, hybrid silicon laser, etc.

A laser may be combined with an upconverter in order to arrive at shorter (laser) wavelengths. For instance, with some (trivalent) rare earth ions upconversion may be obtained or with non-linear crystals upconversion can be obtained. Alternatively, a laser can be combined with a downconverter, such as a dye laser, to arrive at longer (laser) wavelengths.

As can be derived from the below, the term "laser light source" may also refer to a plurality of (different or identical) laser light sources. In specific embodiments, the term "laser light source" may refer to a plurality N of (identical) laser light sources. In embodiments, N=2, or more. In specific embodiments, N may be at least 5, such as especially at least 8. In this way, a higher brightness may be obtained. In embodiments, laser light sources may be arranged in a laser bank (see also above). The laser bank may in embodiments comprise heat sinking and/or optics e.g. a lens to collimate the laser light.

The laser light source is configured to generate laser light source light (or "laser light"). The light source light may essentially consist of the laser light source light. The light source light may also comprise laser light source light of two or more (different or identical) laser light sources. For instance, the laser light source light of two or more (different or identical) laser light sources may be coupled into a light guide, to provide a single beam of light comprising the laser light source light of the two or more (different or identical) laser light sources. In specific embodiments, the light source light is thus especially collimated light source light. In yet further embodiments, the light source light is especially (collimated) laser light source light.

The phrases "different light sources" or "a plurality of different light sources", and similar phrases, may in embodiments refer to a plurality of solid-state light sources selected from at least two different bins. Likewise, the phrases "identical light sources" or "a plurality of same light sources", and similar phrases, may in embodiments refer to a plurality of solid-state light sources selected from the same bin.

The light source is especially configured to generate light source light having an optical axis (O), (a beam shape,) and a spectral power distribution. The light source light may in embodiments comprise one or more bands, having band widths as known for lasers. In specific embodiments, the band(s) may be relatively sharp line(s), such as having full width half maximum (FWHM) in the range of less than 20 nm at RT, such as equal to or less than 10 nm. Hence, the light source light has a spectral power distribution (intensity on an energy scale as function of the wavelength) which may comprise one or more (narrow) bands.

The beams (of light source light) may be focused or collimated beams of (laser) light source light. The term "focused" may especially refer to converging to a small spot. This small spot may be at the discrete converter region, or (slightly) upstream thereof or (slightly) downstream thereof. Especially, focusing and/or collimation may be such that the cross-sectional shape (perpendicular to the optical axis) of the beam at the discrete converter region (at the side face) is essentially not larger than the cross-section shape (perpendicular to the optical axis) of the discrete converter region (where the light source light irradiates the discrete converter region). Focusing may be executed with one or more optics, like (focusing) lenses. Especially, two lenses may be applied to focus the laser light source light. Collimation may be executed with one or more (other) optics, like collimation elements, such as lenses and/or parabolic mirrors.

Hence, in specific embodiments, the light sources may be selected from the group of laser diodes and superluminescent diodes. In other embodiments, the light sources may comprise LEDs. Combinations may also be possible. Therefore, especially the one or more first light sources and the one or more second light sources are solid state light sources.

Especially, the first light source(s) may provide radiation having intensities at shorter wavelengths than the second light source(s). Further, the first light source(s) may provide radiation having intensity at one or more wavelengths equal to or below 420 nm, even more especially at one or more wavelengths equal to or below 380 nm.

It is herein not excluded that the first light source(s) may also provide radiation at one or more wavelengths larger than 380 nm (or even larger than 420 nm). However, especially the light source provides radiation at one or more wavelengths equal to or below 420 nm, such as in the wavelength range of 100-420 nm, even more especially at one or more wavelengths equal to or below 380 nm, such as in the wavelength range of 100-380 nm. This first light source light provided by the one or more first light sources may be defined by the centroid wavelength in the wavelength range of at maximum 420 nm, such as in the wavelength range of 100-420 nm, even more especially at one or more wavelengths equal to or below 380 nm, such as in the wavelength range of 100-380 nm. Herein, the invention is further explained especially in relation to embodiments wherein the first light source light provided by the one or more first light sources may be defined by the centroid wavelength in the wavelength range of at maximum 380 nm.

Hence, in embodiments the one or more first light sources are configured to generate in an operational mode first light source light having a first centroid wavelength (λc1) defined within a wavelength range of at maximum 380 nm.

The term "centroid wavelength", also indicated as λc, is known in the art, and refers to the wavelength value where half of the light energy is at shorter and half the energy is at longer wavelengths; the value is stated in nanometers (nm). It is the wavelength that divides the integral of a spectral power distribution into two equal parts as expressed by the formula λc = Σ λ*I(λ) / (Σ I(λ), where the summation is over the wavelength range of interest, and I(λ) is the spectral energy density (i.e. the integration of the product of the wavelength and the intensity over the emission band normalized to the integrated intensity). The centroid wavelength may e.g. be determined at operation conditions.

Especially, the second light source(s) may provide radiation having intensities at shorter wavelengths than the second light source(s). Further, the second light source(s) may provide radiation having intensity at one or more wavelengths equal to or below 420 nm.

It is herein not excluded that the second light source(s) may also provide radiation at one or more wavelengths larger than 420 nm). However, especially the light source provides radiation at one or more wavelengths equal to or below 420 nm, such as in the wavelength range of 100-420 nm. This second light source light provided by the one or more second light sources may be defined by the centroid wavelength in the wavelength range of at maximum 420 nm, such as in the wavelength range of 100-420 nm. Herein, the invention is further explained especially in relation to embodiments wherein the second light source light provided by the one or more second light sources may be defined by the centroid wavelength in the wavelength range of at maximum 420 nm.

Especially, the one or more second light sources are configured to generate in an operational mode second light source light having a second centroid wavelength (λc2) defined within the wavelength range of at maximum 420 nm.

The phrase "having a second centroid wavelength (λc1) defined within a wavelength range of at maximum 420 nm" may especially indicate that the radiation is generated with at least intensity at wavelengths of 420 nm and smaller, such as in the wavelength range of 100-420 nm, leading to the second centroid wavelength.

In embodiments, wherein a plurality of second light sources are applied, in specific embodiments the plurality of light sources are selected from the same bin or from two bins or from two bins.

Especially, the second centroid wavelength (λc2) > the first centroid wavelength (λc1). Hence, the centroid intensity of the first light source(s) (within the ≤ 380 nm wavelength, like in the wavelength range of 100-380 nm) is at a smaller wavelength than the centroid intensity of the second light source(s) (within the ≤ 420 nm wavelength, like in the wavelength range of 100-420 nm). Especially, in embodiments the second centroid wavelength (λc2) - the first centroid wavelength (λc1) ≥ 5 nm, such as the second centroid wavelength (λc2) - the first centroid wavelength (λc1) ≥ 10 nm, even more especially the second centroid wavelength (λc2) - the first centroid wavelength (λc1) ≥15 nm, like in embodiments the second centroid wavelength (λc2) - the first centroid wavelength (λc1) ≥ 20 nm.

In embodiments, the one or more first light sources may be configured to generate in an operational mode first light source light having a first centroid wavelength (λc1) defined within a wavelength range of at maximum 340 nm (such as selected from the range of 100-340 nm, like 190-340 nm), and the one or more second light sources may be configured to generate in an operational mode second light source light having a second centroid wavelength (λc2) defined within the wavelength range of at maximum 380 nm (i.e. UV light) (such as selected from the range of 100-380 nm, like 190-380 nm), wherein the second centroid wavelength (λc2) > the first centroid wavelength (λc1), especially wherein the second centroid wavelength (λc2) - the first centroid wavelength (λc1) ≥ 5 nm, such as the second centroid wavelength (λc2) - the first centroid wavelength (λc1) ≥ 10 nm, even more especially the second centroid wavelength (λc2) - the first centroid wavelength (λc1) ≥ 15 nm, like in embodiments the second centroid wavelength (λc2) - the first centroid wavelength (λc1) ≥ 20 nm. In specific embodiments, the second centroid wavelength (λc2) - the first centroid wavelength (λc1) ≥ 30 nm, like the second centroid wavelength (λc2) - the first centroid wavelength (λc1) ≥ 40 nm.

In embodiments, the one or more first light sources may be configured to generate in an operational mode first light source light having a first centroid wavelength (λc1) defined within a wavelength range of at maximum 315 nm (i.e. UV-C and/or UV-B light) (such as selected from the range of 100-315 nm, like 190-315 nm), and the one or more second light sources may be configured to generate in an operational mode second light source light having a second centroid wavelength (λc2) defined within the wavelength range of at maximum 365 nm (such as selected from the range of 100-365 nm, like 190-365 nm), wherein the second centroid wavelength (λc2) > the first centroid wavelength (λc1), especially wherein the second centroid wavelength (λc2) -the first centroid wavelength (λc1) ≥ 5 nm, such as the second centroid wavelength (λc2) - the first centroid wavelength (λc1) ≥ 10 nm, even more especially the second centroid wavelength (λc2) -the first centroid wavelength (λc1) ≥ 15 nm, like in embodiments the second centroid wavelength (λc2) - the first centroid wavelength (λc1) ≥ 20 nm.

In embodiments, the one or more first light sources may be configured to generate in an operational mode first light source light having a first centroid wavelength (λc1) defined within a wavelength range of at maximum 280 nm (i.e. UV-C light) (such as selected from the range of 100-280 nm, like 190-280 nm), and the one or more second light sources may be configured to generate in an operational mode second light source light having a second centroid wavelength (λc2) defined within the wavelength range of at maximum 315 nm (i.e. UV-C and/or UV-B light) (such as selected from the range of 100-315 nm, like 190-315 nm), wherein the second centroid wavelength (λc2) > the first centroid wavelength (λc1), especially wherein the second centroid wavelength (λc2) - the first centroid wavelength (λc1) ≥ 5 nm, such as the second centroid wavelength (λc2) - the first centroid wavelength (λc1) ≥ 10 nm, even more especially the second centroid wavelength (λc2) - the first centroid wavelength (λc1) ≥ 15 nm, like in embodiments the second centroid wavelength (λc2) - the first centroid wavelength (λc1) ≥ 20 nm.

Especially, the first light source light may be provided as narrower beam, which may be indicated as first beam, and the second light source light may be provided as broader beam, indicated as second beam. Hence, in embodiments (in an operational mode (of the radiation generating system)) the radiation generating system may be configured to provide a beam of radiation comprising one or more of (i) the first light source light and (ii) the second light source light; wherein the first light source light has a first beam angle (α1), wherein the second light source light has a second beam angle (α2), and wherein the first beam angle (α1) < the second beam angle (α2).

Hence, in embodiments in an operational mode of the radiation generating system a beam of radiation is provided comprising one or more of (i) the first beam comprising first light source light having the first beam angle (α1) defined by full width half maxima and (ii) the second beam comprising second light source light having s the second beam angle (α2) defined by full width half maxima; wherein the first beam and the second beam at least partly overlap, and wherein the first beam angle (α1) < the second beam angle (α2).

The first beam may have a first optical axis and the second beam may have a second optical axis. Especially, the optical axis of the first beam and the optical axis of the second beam may have a mutual angle αm, wherein 0° ≤ αm < α2 - α1. Especially, in embodiments, the mutual angle may be essentially zero degrees. Especially, the first light source light and the second light source light are provided in essentially the same direction. Hence, the first beam and the second beam at least partly overlap. Even more especially, one of the first beam and the second beam may essentially overlap with the other of the first beam and the second beam. Especially, the first beam may essentially overlap with the (broader) second beam; the second beam may partly overlap with the (narrower) first beam. Yet further, in embodiments the optical axis of the first beam and the second beam may essentially coincide. Hence, the first light and the second light may propagate in the same direction and at least partly overlap.

Note that in embodiments in an operational mode the first light source light and the second light source light may be provided simultaneously. However, alternatively or additionally, in other embodiments mode the first light source light and the second light source light may be controllable in such a way, that they may be provided simultaneously or may be provided not simultaneously (see further also below).

In embodiments, the first light sources may provide the more effective radiation, and the second light sources may provide the less harmful radiation. Hence, the first centroid wavelength (λc1) may in embodiments be selected within the wavelength range of 100-280 nm, especially within the wavelength range of 190-280 nm, more especially within the wavelength range of 190-230 nm. Additionally or alternatively, the second centroid wavelength (λc2) may be selected within the wavelength range of 280-420 nm. In specific embodiments, the second centroid wavelength (λc2) - the first centroid wavelength (λc1) ≥5 nm, such as the second centroid wavelength (λc2) - the first centroid wavelength (λc1) ≥10 nm, like even more especially the second centroid wavelength (λc2) - the first centroid wavelength (λc1) ≥ 15 nm. In yet more specific embodiments, the second centroid wavelength (λc2) - the first centroid wavelength (λc1) ≥ 20 nm, such as the second centroid wavelength (λc2) - the first centroid wavelength (λc1) ≥ 30 nm, like the second centroid wavelength (λc2) - the first centroid wavelength (λc1) ≥ 40 nm.

The first beam angle (α1) may in embodiments be selected from the range of at maximum 20°, like at maximum 15°, such as at maximum 10°. Additionally or alternatively, the first beam angle (α1) may in embodiments be selected from the range of at least 1.5°, such as at least 2°, like at least 5°. Additionally or alternatively, the second beam angle (α2) may in embodiments be selected from the range of at least 5°, such as at least 10°, like at least 15°. Alternatively or additionally, the second beam angle (α2) may in embodiments be selected from the range of at maximum 100°, such as at maximum 90°. Even more especially, the second beam angle (α2) may in embodiments be selected from the range of at maximum 75°, such as at maximum 60°. Yet even more especially, the second beam angle (α2) may in embodiments be selected from the range of at maximum 50°, such as at maximum 40°, such as in specific embodiments at maximum 30°. However, other values of the beam angles (based on full width half maxima are herein not excluded).

Especially, however in embodiments the FWHM of the first light source light is more narrow than of the second light source light. Especially, in embodiments the second beam angle (α2) - the first beam angle (α1) ≥ 3°, especially the second beam angle (α2) - the first beam angle (α1) ≥ 5°, more especially the second beam angle (α2) - the first beam angle (α1) ≥ 10°.

Especially, the radiation generating system may be configured to provide the beam of radiation in an operational mode comprising both the first light source light and the second light source light.

Hence, in specific embodiments, the first centroid wavelength (λc1) is selected within the wavelength range of 100-280 nm, wherein the second centroid wavelength (λc2) is selected within the wavelength range of 280-420 nm, and wherein the second centroid wavelength (λc2) - the first centroid wavelength (λc1) ≥ 30 nm; wherein the first beam angle (α1) is selected from the range of at maximum 15°, wherein the second beam angle (α2) is selected from the range of at least 10°, wherein the second beam angle (α2) - the first beam angle (α1) ≥ 5°; and wherein the radiation generating system is configured to provide the beam of radiation in an operational mode comprising both the first light source light and the second light source light.

In embodiments, the beam provided by the first light source light may have essentially the same cross-sectional shape as the beam provided by the second light source light. For instance, both may have an essentially round cross-section. In other embodiment, both may have another type of cross-sectional shape, like oval or elliptical. The cross-section may be defined by the intensity at full width half maximum. The beams may also have different cross-sectional shapes. At least along one axis in the cross-sectional shapes, or along two orthogonal axes in the cross-sectional shapes may apply the second beam angle (α2) - the first beam angle (α1) ≥ 3°, especially the second beam angle (α2) - the first beam angle (α1) ≥ 5°, more especially the second beam angle (α2) - the first beam angle (α1) ≥ 10°. In specific embodiments, the second beam angle (α2) - the first beam angle (α1) ≤ 75°, such as especially in embodiments the second beam angle (α2) - the first beam angle (α1) ≤ 60°, like in specific embodiments the second beam angle (α2) -the first beam angle (α1) ≤ 50°. Other values, however, are herein not excluded.

The radiation generating system may have an optical axis (O). In embodiments, the one or more first light sources may be configured closer to the optical axis (O) than the one or more second light sources. The phrase "first light sources may be configured closer to the optical axis (O)" and similar phrases may especially indicate that the first light sources may be configured closer to the optical axis, or an elongation thereof. When being configure closer to the optical axis, a downstream configured optical element (see further also below) may better beam shape the first light source light than the second light source light when the second light sources are configured further away from the optical axis.

Hence, additionally or alternatively, in embodiments, the radiation generating system may comprise a plurality of second light sources.

In specific embodiments, the one or more first light sources and the plurality of second light sources may be configured in an array. The array may have an array center. Especially, the one or more first light sources may have a first average distance (dₐ₁) to array center, and the plurality of second light sources have a second average distance (dₐ₂) to array center. In specific embodiments, the first average distance (dₐ₁) < the second average distance (da2).

Hence, in specific embodiments, the radiation generating system comprises a plurality of second light sources, wherein the one or more first light sources and the plurality of second light sources are configured in an array, wherein the array has an array center, wherein the one or more first light sources have a first average distance (dₐ₁) to array center, and wherein the plurality of second light sources have a second average distance (dₐ₂) to array center, wherein the first average distance (dₐ₁) < the second average distance (dₐ₂). In this way, the more effective radiation may be specifically located close to the center of the beam of radiation. For instance, in embodiments the first average distance (dₐ₁) / the second average distance (dₐ₂) ≤ 0.9, such as in embodiments the first average distance (dₐ₁) / the second average distance (dₐ₂) ≤ 0.8. For instance, in embodiments 0.2 ≤ the first average distance (dₐ₁) / the second average distance (dₐ₂) ≤ 0.75.

As indicated above, the radiation generation system may comprise first light sources and second light source. In embodiments, the first light sources may be selected out of a number of options. Yet, in embodiments the second light sources may be selected out of a number of options. Even, in embodiments the number of options may partly over. Hence, first light sources in one embodiment may be used as second light sources in other embodiments and/or second light sources in one embodiment may be used as first light sources in other embodiments. However, as indicated above, especially in embodiments the first lights sources and the second light sources may be selected such that the second centroid wavelength (λc2) > the first centroid wavelength (λc1). Further, the radiation generating system may especially be configured such that the first beam angle (α1) < the second beam angle (α2).

In specific embodiments, the first centroid wavelength (λc1) may be selected within the wavelength range of 230-280 nm and the second centroid wavelength (λc2) may be selected within the wavelength range of 280-315 nm; with especially the second centroid wavelength (λc2) - the first centroid wavelength (λc1) ≥ 15 nm (see also above).

In embodiments, the one or more first light sources and the one or more second light sources of the radiation generating system may comprise one or more of, especially two or more of (a) a number n11 of far UV-C light sources configured to generate far UV-C light having a centroid wavelength (λ₁₁) within the wavelength range of at maximum 230 nm. Alternatively or additionally, in embodiments the radiation generating system may comprise a number n12 of near UV-C light sources configured to generate near UV-C light having a centroid wavelength (λ₁₂) within the wavelength range of 230-280 nm. Alternatively or additionally, in embodiments the radiation generating system may comprise a number n23 of UV-B light sources configured to generate UV-B light having a centroid wavelength (λ₂₃) selected from the wavelength range of 280-315 nm. Alternatively or additionally, in embodiments the radiation generating system may comprise a number n24 of UV-A light sources configured to generate UV-A light having a centroid wavelength (λ₂₄) selected from the wavelength range of 315-380 nm. Alternatively or additionally, in embodiments the radiation generating system may comprise a number n25 0f violet light sources configured to generate violet light having a centroid wavelength (λ₂₅) selected from the wavelength range of 380-420 nm. Especially, the centroid wavelength (λ11) < the centroid wavelength (λ12) < the centroid wavelength (λ23) < the centroid wavelength (λ24) < the centroid wavelength (λ25). For instance, in embodiments the centroid wavelength (λ12) - the centroid wavelength (λ11) ≥ 5 nm, even more especially at least about 10 nm, such as even more especially at least about 15 nm, such as even at least about 20 nm. For instance, in embodiments the centroid wavelength (λ21) - the centroid wavelength (λ12) ≥ 5 nm, even more especially at least about 10 nm, such as even more especially at least about 15 nm, such as even at least about 20 nm. For instance, in embodiments the centroid wavelength (λ22) - the centroid wavelength (λ21) ≥ 5 nm, even more especially at least about 10 nm, such as even more especially at least about 15 nm, such as even at least about 20 nm. For instance, in embodiments the centroid wavelength (λ23) - the centroid wavelength (λ22) ≥ 5 nm, even more especially at least about 10 nm, such as even more especially at least about 15 nm, such as even at least about 20 nm. As can be derived from the above "light sources" may also be indicated as "radiation sources" or "sources of radiation".

Hence, in specific embodiments the radiation generating system may comprise two or more of (a) a number n11 of far UV-C light sources configured to generate far UV-C light having a centroid wavelength within the wavelength range of at maximum 230 nm, (b) a number n12 of near UV-C light sources configured to generate near UV-C light having a centroid wavelength within the wavelength range of 230-280 nm, (c) a number n23 of UV-B light sources configured to generate UV-B light having a centroid wavelength selected from the wavelength range of 280-315 nm, (d) a number n24 of UV-A light sources configured to generate UV-A light having a centroid wavelength selected from the wavelength range of 315-380 nm, and (e) a number n25 of violet light sources configured to generate violet light having a centroid wavelength selected from the wavelength range of 380-420 nm. As can be derived from the above, far UV-C light may also be indicated as far UV-C radiation, near UV-C light may be indicated as near UV-C radiation, UV-B light may be indicated as UV-B radiation, UV-A light may be indicated as UV-A radiation, and violet light may be indicated as violet radiation.

Hence, in embodiments the one or more first light source light may comprise one or more far UV-C light and near UV-C light. Further, in embodiments the one or more second light source light may comprise one or more of UV-B light, UV-A light, and violet light, even more especially one or more of UV-B light and UV-A light.

Hence, in embodiments the one of more first light sources may comprise one or more of a number n11 of far UV-C light sources and (n12) near UV-C light sources. Further, in embodiments the one or more second light sources may comprise one or more of a number n23 of UV-B light sources, a number n24 of UV-A light sources, and a number n25 of violet light sources, especially one or more of a number n23 of UV-B light sources and a number n24 of UV-A light sources.

Hence, in embodiments in an operational mode of the radiation generating system a beam of radiation is provided comprising one or more of (i) a far UV-C light beam comprising far UV-C light having a far UV-C light beam angle (α11) defined by full width half maxima, (ii) a near UV-C light beam comprising near UV-C light having a near UV-C light beam angle (α12) defined by full width half maxima, (iii) a UV-B light beam comprising UV-B light having a UV-B light beam angle (α23) defined by full width half maxima, (iv) a UV-A light beam comprising UV-A light having a UV-A light beam angle (α24) defined by full width half maxima, and (v) a violet light beam comprising violet light having a violet light beam angle (α25) defined by full width half maxima, wherein especially any combination of two beams at least partly overlaps.

Each of the beams may have an optical axis. In embodiments, any combination of two beams may have a mutual angle αₘ, wherein 0° ≤ αₘ < |αₓ₁-αₓ₂|, wherein αₓ₁ indicates the beam angle of one of the beams and αₓ₂ the beam angle of the other of the beams.

Especially, the far UV-C light, near UV-C light, UV-B light, UV-A light, and violet light are provided in essentially the same direction. Hence, any combination of two beams of far UV-C light, near UV-C light, UV-B light, UV-A light, and violet light may at least partly overlap. Even more especially, one of the beams of far UV-C light, near UV-C light, UV-B light, UV-A light, and violet light may essentially overlap with another one of the beams of far UV-C light, near UV-C light, UV-B light, UV-A light, and violet light. Yet further, in embodiments the optical axes of two or more, especially three or more, even more especially four or more, yet even more especially of all beams of far UV-C light, near UV-C light, UV-B light, UV-A light, and violet light may essentially coincide. Hence, the far UV-C light, near UV-C light, UV-B light, UV-A light, and violet light may propagate in the same direction and at least partly overlap.

Especially, in embodiment the radiation generating system comprises at least one of the far UV-C light sources and the near UV-C light sources. In embodiments, the radiation generating system comprises both the far UV-C light sources and the near UV-C light sources. Especially, in (other) embodiment the radiation generating system comprises (a) at least one of the far UV-C light sources and the near UV-C light sources, and (b) at least one of the UV-B light sources, the UV-A light sources, and the violet light sources. Especially, in (other) embodiment the radiation generating system comprises (a) far UV-C light sources and (b) at least one of the near UV-C light sources, the UV-B light sources, the UV-A light sources, and the violet light sources. Especially, in (other) embodiment the radiation generating system comprises (a) near UV-C light sources, and (b) at least one of the UV-B light sources, the UV-A light sources, and the violet light sources.

In specific embodiments, the number n11 + the number n12 ≥ 1. Alternatively or additionally, in specific embodiments the number n23 + the number n24 + the number n25 ≥ 1. More especially, in embodiments the number 23 + the number n24 + the number n25 ≥ 2. Hence, especially in embodiments the number n11 ≥ 0; the number n12 ≥ 0; the number n23 ≥ 0; the number n24 ≥ 0; the number n25 ≥ 0; the number n11 + n12 ≥ 1 and wherein the number n23 + the number n24 + the number n25 ≥ 1, even more especially the number n23 + the number n24 + the number n25 ≥ 2.

In embodiments, the radiation generating system comprises less far UV-C light sources than near UV-C light sources, but especially comprises at least both, and may further comprise violet light sources. Hence, in embodiments the number n11 < the number n12 and the number n25 ≥ 1. When the light sources are configured in an array, especially the violet light sources may be configured most remote from a center of the array.

As indicated above, in embodiments the first light sources may comprise one or two types of light sources. Further, in embodiments the second light source may comprise one, two, or three different types of light sources. Further types, however, are herein not excluded. Hence, in embodiments the radiation generating system may comprise two types of light sources, or three types of light sources, or in specific embodiments four types of light sources, or even five types of light sources. Especially, the beam angle may in embodiments increase with increasing centroid wavelength.

In embodiments, the radiation generating system may be configured to provide the beam of radiation comprising: (i) near UV-C light having a beam angle (α12), (ii) UV-B light having a beam angle (α23), and (iii) UV-A light having a beam angle (α24) and/or violet light having a beam angle (α25). Especially, in embodiments the beam angle (α12) < the beam angle (α23) < the beam angle (α24) and/or the beam angle (α12) < the beam angle (α23) < the beam angle (α25). Further, in specific embodiments the number (n12) ≥ 1;the number (n23) ≥ 1; the number n24 + the number n25 ≥ 1.

In (alternative) embodiments, the radiation generating system may be configured to provide the beam of radiation comprising: (i) near UV-C light having a beam angle (α12), (ii) far UV-C light having a beam angle (α11), and (iii) one or more of UV-B light having a beam angle (α23), UV-A light having a beam angle (α24) and violet light having a beam angle (α25). Especially, in embodiments the beam angle (α12) < the beam angle (α11) < the beam angle (α23) and/or the beam angle (α12) < the beam angle (α11) < the beam angle (α24) and/or the beam angle (α12) < the beam angle (α11) < the beam angle (α25). Further, in specific embodiments the number (n11) ≥ 1; the number (n12) ≥ 1; the number (n24) + the number (n23) + the number (n25) ≥ 1.

Phrases like "the radiation generating system may be configured to provide the beam of radiation comprising (i) near UV-C light, (ii) UV-B light, and (iii) UV-A light and/or violet light" or "the radiation generating system may be configured to provide the beam of radiation comprising (i) near UV-C light, (ii) far UV-C light, and (iii) one or more of UV-B light, UV-A light and violet light", and similar phrases may especially indicate that in an operational mode such beam is provided, but does not exclude other operational modes with less or more components. For instance, when the light sources are controllable, the radiation generating system of the former embodiment may in another operational mode also be configured to provide the beam of radiation only comprising near UV-C light, or only comprising UV-B light, or comprising only a combination of these, or only comprising (iii) UV-A light, or comprising a combination of UV-A light and violet light, etc.

In embodiments, at least one or more of the following may apply the beam angle (α11) < the beam angle (α23), the beam angle (α11) < the beam angle (α24), the beam angle (α11) < the beam angle (α25), the beam angle (α12) < the beam angle (α23), the beam angle (α12) < the beam angle (α24), and the beam angle (α12) < the beam angle (α25). Further, especially the beam angle (α25) is larger than any of the other beam angles.

As indicated above, a beam provided one of the first type of light sources may have essentially the same cross-sectional shape as a beam provided by one of the second type of light sources. For instance, both may have an essentially round cross-section. In other embodiment, both may have another type of cross-sectional shape, like oval or elliptical. The cross-section may be defined by the intensity at full width half maximum. The beams may also have different cross-sectional shapes. At least along one axis in the cross-sectional shapes, or along two orthogonal axis in the cross-sectional shapes at least one or more of the following may apply: (i) the beam angle (α23) - the beam angle (α11) ≥ 3°, especially the beam angle (α23) - the beam angle (α11) ≥ 5°, more especially the beam angle (α11) ≥ 10°, (ii) the beam angle (α24) - the beam angle (α11) ≥ 3°, especially the beam angle (α24) - the beam angle (α11) ≥ 5°, more especially the beam angle (α24) - the beam angle (α11) ≥ 10°, (iii) the beam angle (α25) - the beam angle (α11) ≥ 3°, especially the beam angle (α25) - the beam angle (α11) ≥ 5°, more especially the beam angle (α25) - the beam angle (α11) ≥ 10°, (iv) the beam angle (α23) - the beam angle (α12) ≥ 3°, especially the beam angle (α23) - the beam angle (α12) ≥ 5°, more especially the beam angle (α23) - the beam angle (α12) ≥ 10°, (v) the beam angle (α24) - the beam angle (α12) ≥ 3°, especially the beam angle (α24) - the beam angle (α12) ≥ 5°, more especially the beam angle (α24) - the beam angle (α12) ≥ 10°, and (vi) the beam angle (α25) - the beam angle (α12) ≥ 3°, especially the beam angle (α25) - the beam angle (α12) ≥ 5°, more especially the beam angle (α25) - the beam angle (α12) ≥ 10°.

In embodiments, the radiation generating system may be configured to provide in an operational mode the beam of radiation comprising at least two of (i) far UV-C light, (ii) near UV-C light, (iii) UV-B light, (iv) UV-A light, and (v) violet light.

In specific embodiments, in an operational mode (of the radiation generating system (or disinfection device; see also below), the first light source light and the second light source light may have differently shaped cross-sections, wherein one of the first light source light and the second light source light may provide a circular cross-sectional beam shape and the other of the first light source light and the second light source light may provide a non-circular cross-sectional beam shape.

As indicated above, the light sources may be configured in an array, especially a 2D array. Such array may comprise light sources configured closer to a center of the array and light sources configured closer to an edge of the array. Especially, first light sources may be configured closer to a center of the array and second light sources may be configured closer to an edge of the array. When using e.g. violet light sources as outer light sources, this may provide a violet halo effect. This may be useful to mark the beam with its useful properties. Hence, in embodiments the array may comprise an array edge, wherein a majority of the light sources (110,120) defining the array edge may be selected from the group of violet light sources. In specific embodiments, the larger the centroid wavelength, the further from the array center. In embodiments, the array may comprise at least 7 light sources, such as at least 12 light sources.

Hence, in embodiments the radiation generating system may comprise two or more of (a) far UV-C light sources, (b) near UV-C light sources, (c) UV-B light sources, (d) UV-A light sources, and (e) violet light sources configured to generate violet light, wherein a majority of the light sources (110,120) defining the array edge may be selected from the group of violet light sources.

In other embodiments, the radiation generating system may comprise two or more of (a) far UV-C light sources, (b) near UV-C light sources, (c) UV-B light sources, and (d) UV-A light sources, wherein a majority of the light sources (110,120) defining the array edge may be selected from the group of UV-A light sources.

In embodiments, the radiation generating system may further comprise a control system. Especially, the control system may be configured to control one or more of a spectral power distribution of the system light and a spatial power distribution of the system light. Especially, the control system may be configured to simultaneously control the spectral power distribution of the system light and the spatial power distribution of the system light.

In embodiments, the control system may be configured to control the system light from a (first operational mode with a) first spectral power distribution and a first spatial power distribution to a (second operational mode with a) second spectral power distribution different from said first spectral power distribution and a second spatial power distribution different from said first spatial power distribution. For instance, in the first operational mode the relative contribution of first light source light to the system light may be larger than the relative contribution of the second light source light to the system light, and in the second operational mode the relative contribution of first light source light to the system light may be smaller than the relative contribution of the second light source light to the system light.

Especially, in embodiments each type of light source may be controlled individually. Further, a type of light sources may be divided in two or more subsets of such type of light sources, like a subset of that type of light sources closer to a center of an array and another subset of that same type of light sources configured surrounding the former subset. Hence, in specific embodiments the control system is configured to individually control two or more, such as especially three or more of the (a) a number n11 of far UV-C light sources, (b) a number n12 of near UV-C light sources, (c) a number n23 of UV-B light sources, (d) a number of n24 UV-A light sources, and (e) a number n25 of violet light sources.

Note that all five different types of light sources may be comprised by the radiation generating system, but also less types may be available. For instance, with reference to one of the embodiments described above, the radiation generating system may be configured to provide the beam of radiation comprising (i) near UV-C light, (ii) UV-B light, and (iii) UV-A light and/or violet light, and the control system may be configured to individually control (a) the number n12 of near UV-C light sources, (b) the number n23 of UV-B light sources, and (c) the number n24 of UV-A light sources and/or the number n25 of violet light sources. See also above for further specific embodiments. Controlling light sources may especially refer to controlling their intensity, such as via pulse width modulation, and at least controlling on and off states.

As indicated above, in embodiments, the control system may be configured to control the system light from a (first operational mode with a) first spectral power distribution and a first spatial power distribution to a (second operational mode with a) second spectral power distribution different from said first spectral power distribution and a second spatial power distribution different from said first spatial power distribution. For instance, in the first operational mode the relative contribution of one or more of the far UV-C light, near UV-C light, UV-B light, UV-A light, violet light to the system light may be larger than one or more other(s) of the far UV-C light, near UV-C light, UV-B light, UV-A light, violet light, and in the second operational mode the relative contribution of one or more of the far UV-C light, near UV-C light, UV-B light, UV-A light, violet light to the system light may be smaller than one or more other(s) of the far UV-C light, near UV-C light, UV-B light, UV-A light, violet light.

In specific embodiments, the control system may be configured to simultaneously control a spectral power distribution of the system light and a spatial power distribution of the system light; wherein the control system is configured to individually control three or more of the (a) a number n11 of far UV-C light sources, (b) a number n12 of near UV-C light sources, (c) a number n23 of UV-B light sources, (d) a number n24 of UV-A light sources, and (e) a number n25 of violet light sources.

By individually control two or more, especially three or more, of the (a) a number n11 of far UV-C light sources, (b) a number n12 of near UV-C light sources, (c) a number n23 of UV-B light sources, (d) a number n24 of UV-A light sources, and (e) a number n25 of violet light sources, the spectral power distribution and the spatial power distribution of the system light may be controlled.

The term "controlling" and similar terms especially refer at least to determining the behavior or supervising the running of an element. Hence, herein "controlling" and similar terms may e.g. refer to imposing behavior to the element (determining the behavior or supervising the running of an element), etc., such as e.g. measuring, displaying, actuating, opening, shifting, changing temperature, etc.. Beyond that, the term "controlling" and similar terms may additionally include monitoring. Hence, the term "controlling" and similar terms may include imposing behavior on an element and also imposing behavior on an element and monitoring the element. The controlling of the element can be done with a control system, which may also be indicated as "controller". The control system and the element may thus at least temporarily, or permanently, functionally be coupled. The element may comprise the control system. In embodiments, the control system and element may not be physically coupled. Control can be done via wired and/or wireless control. The term "control system" may also refer to a plurality of different control systems, which especially are functionally coupled, and of which e.g. one control system may be a master control system and one or more others may be slave control systems. A control system may comprise or may be functionally coupled to a user interface.

The control system may also be configured to receive and execute instructions form a remote control. In embodiments, the control system may be controlled via an App on a device, such as a portable device, like a smartphone e.g. an iPhone, a tablet, etc.. The device is thus not necessarily coupled to the lighting system, but may be (temporarily) functionally coupled to the lighting system.

Hence, in embodiments the control system may (also) be configured to be controlled by an App on a remote device. In such embodiments the control system of the lighting system may be a slave control system or control in a slave mode. For instance, the lighting system may be identifiable with a code, especially a unique code for the respective lighting system. The control system of the lighting system may be configured to be controlled by an external control system which has access to the lighting system on the basis of knowledge (input by a user interface of with an optical sensor (e.g. QR code reader) of the (unique) code. The lighting system may also comprise means for communicating with other systems or devices, such as on the basis of Bluetooth, WIFI, LiFi, ZigBee, BLE or WiMAX, or another wireless technology.

The system, or apparatus, or device may execute an action in a "mode" or "operation mode" or "mode of operation" or "operational mode". Likewise, in a method an action or stage, or step may be executed in a "mode" or "operation mode" or "mode of operation" or "operational mode". This does not exclude that the system, or apparatus, or device may also be adapted for providing another controlling mode, or a plurality of other controlling modes. Likewise, this may not exclude that before executing the mode and/or after executing the mode one or more other modes may be executed.

However, in embodiments a control system may be available, that is adapted to provide at least the controlling mode. Would other modes be available, the choice of such modes may especially be executed via a user interface, though other options, like executing a mode in dependence of a sensor signal or a (time) scheme, may also be possible. The operation mode may in embodiments also refer to a system, or apparatus, or device, that can only operate in a single operation mode (i.e. "on", without further tunability).

Hence, in embodiments, the control system may control in dependence of one or more of an input signal of a user interface, a sensor signal (of a sensor), and a timer. The term "timer" may refer to a clock and/or a predetermined time scheme.

Hence, the radiation generating system may also comprise or be functionally coupled to a sensor. The term "sensor" may also refer to a plurality of (different) sensors. In embodiments, the sensor may comprise one or more sensors selected from the group comprising: a movement sensor, a presence sensor, a distance sensor, an ion sensor, a gas sensor, a volatile organic compound sensor, a pathogen sensor, an airflow sensor, a sound sensor, and a communication receiver. The pathogen sensor may comprise a sensor for one or more of bacteria, viruses, and spores. Alternatively or additionally, the sensor may comprise a temperature sensor. Further, alternatively or additionally, the sensor may comprise a humidity sensor. The control system may be functionally coupled to this sensor.

The control system may be configured to individually control the one or more first light sources and the one or more second light sources in dependence of a sensor signal of the sensor. Hence, in specific embodiments the control system may be configured to individually control two or more of the (a) a number n11 of Far UV-C light sources, (b) a number n12 of near UV-C light sources, (c) a number n23 of UV-B light sources, (d) a number n24 of UV-A light sources, and (e) a number n25 of violet light sources in dependence of a sensor signal of the sensor.

By controlling the light sources the spectral power distribution of the system light may be controlled and/or the spatial power distribution of the system light may be controlled. For instance, in dependence of the light sources that are operated, the beam shape may be controlled (spatial power distribution).

Especially, the radiation generating system may comprising a beam shaping element, wherein the beam shaping element may in embodiments comprises one or more of a collimator, a reflector, a lens, a lens array. In embodiments, the beam shaping element may define the optical axis of the radiation generating system. Especially, the beam shaping elements and light sources may be configured such that the one or more first light sources provide the first light source light with a more collimated beam than the one or more second light sources, i.e. the beam shaping element, the one or more first light sources and the one or more second light sources are configured such that the beam of first light source light is more collimated than the beam of second light source light. Especially, the beam shaping element is configured downstream of the first light sources and/or second light sources. Especially, the beam shaping element is configured downstream of all the light sources that provide the beam of radiation.

The terms "upstream" and "downstream" relate to an arrangement of items or features relative to the propagation of the light from a light generating means (here the especially the light source), wherein relative to a first position within a beam of light from the light generating means, a second position in the beam of light closer to the light generating means is "upstream", and a third position within the beam of light further away from the light generating means is "downstream".

In embodiments, the radiation system may comprise a plurality of units, wherein each unit comprises (i) one of more first light sources, (ii) one or more second light sources, and (iii) a beam shaping element configured downstream of the one of more first light sources, one or more second light sources, wherein each unit is configured to provide a beam or beamlet) of radiation comprising one or more of (i) the first light source light and (ii) the second light source light; wherein the first light source light has a first beam angle (α1), wherein the second light source light has a second beam angle (α2), and wherein the first beam angle (α1) < the second beam angle (α2).

In yet a further aspect, the invention provides a disinfection device comprising the light generating system as defined herein. In embodiments, the one or more first light sources may comprise one or more solid state light sources, especially selected from the group of LEDs and superluminescent diodes. In embodiments, alternatively or additionally, the one or more second light sources may comprise one or more solid state light sources, especially selected from the group of LEDs and superluminescent diodes. In specific embodiments, the one or more first light sources and the one or more second light sources may comprise one or more of LEDs and superluminescent diodes.

In embodiments, the disinfection may be provided as spot light. For instance, the beam may have a maximum beam angle of at maximum about 60°. The beam angle may in embodiments be selected from the range of at maximum 50°, such as at maximum 40°, such as in specific embodiments at maximum 30°. Note that the beam angle of the beam of radiation may in embodiments especially be defined by the radiation component having the broadest beam angle.

Therefore, in embodiments the disinfection device (or the radiation generating system) may have the shape of a spotlight.

In yet a further aspect, the invention also provides a method for treating a gas or a surface in a space external from the radiation generating system or the disinfection device as described herein. Especially, the method may comprise providing the radiation to the gas or the surface with the radiation generating system or the disinfection device.

The term "space" may for instance relate to a (part of) hospitality area, such as a restaurant, a hotel, a clinic, or a hospital, etc.. The term "space" may also relate to (a part of) an office, a department store, a warehouse, a cinema, a church, a theatre, a library, etc. However, the term "space" also relate to (a part of) a working space in a vehicle, such as a cabin of a truck, a cabin of an air plane, a cabin of a vessel (ship), a cabin of a car, a cabin of a crane, a cabin of an engineering vehicle like a tractor, etc.. The term "space" may also relate to (a part of) a working space, such as an office, a (production) plant, a power plant (like a nuclear power plant, a gas power plant, a coal power plant, etc.), etc. For instance, the term "space" may also relate to a control room, a security room, etc.. Especially, the term "space" may herein refer to an indoor space. In yet other embodiments, the term "space" may also relate to a toilet room or bathroom. In yet other embodiments, the term "space" may also relate to an elevator. In embodiments, the term "space" may also refer to a conference room, a school room, an indoor hallway, an indoor corridor, an indoor space in an elderly home, an indoor space in a nursing home, etc. In embodiments, the term "space" may refer to an indoor sport space, like a gym, a gymnastics hall, in indoor ball sport space, a ballet room, a swimming pool, a changing room, etc. In embodiments, the term "space" may refer to an (indoor) bar, an (indoor) disco, etc.

As indicated above, the spectral power distribution and/or the spatial power distribution of the beam of radiation of the radiation generating system may be controlled in dependence of a sensor signal.

The terms "visible", "visible light" or "visible emission" and similar terms refer to light having one or more wavelengths in the range of about 380-780 nm. Herein, UV may especially refer to a wavelength selected from the range of 200-380 nm. The terms "light" and "radiation" are herein interchangeably used, unless clear from the context that the term "light" only refers to visible light. In specific embodiments, especially for lighting applications, the terms "light" and "radiation" refer to (at least) visible light. The terms "violet light" or "violet emission" especially relates to light having a wavelength in the range of about 380-440 nm.

In yet a further aspect, the invention also provides a lamp or a luminaire comprising the radiation generating system as defined herein. The luminaire may further comprise a housing, optical elements, louvres, etc. etc. The lamp or luminaire may further comprise a housing enclosing the light generating system. The lamp or luminaire may comprise a light window in the housing or a housing opening, through which the system light may escape from the housing. The light generating device may comprise a housing or a carrier, configured to house or support, one or more elements of the light generating system.

### BRIEF DESCRIPTION OF THE DRAWINGS

Embodiments of the invention will now be described, by way of example only, with reference to the accompanying schematic drawings in which corresponding reference symbols indicate corresponding parts, and in which:
Figs. 1a-1c schematically depict some aspects and embodiments;
Figs. 2a-2c schematically depict some aspects and embodiments; and
Fig. 3 schematically depict some (application) embodiments.

The schematic drawings are not necessarily to scale.

### DETAILED DESCRIPTION OF THE EMBODIMENTS

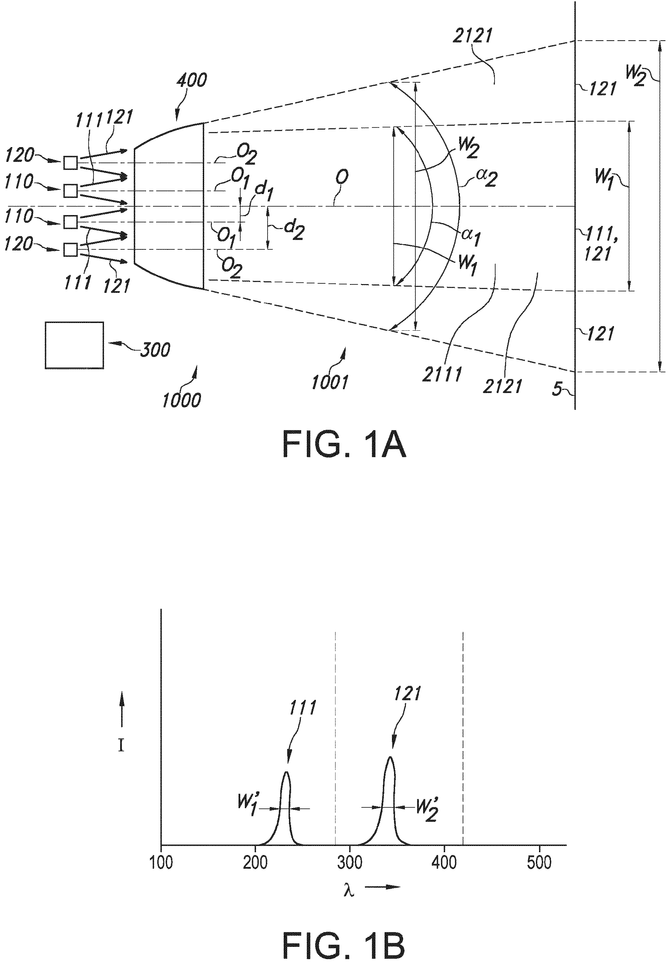

Fig. 1a schematically depicts a radiation generating system 1000 comprising one or more first light sources 110 and one or more second light sources 120. The one or more first light sources 110 and the one or more second light sources 120 are solid state light sources. In embodiments, the one or more first light sources 110 and the one or more second light sources 120 may comprise one or more of LEDs and superluminescent diodes. The radiation generating system 1000 is configured to provide a beam of radiation 1001 comprising one or more of (i) the first light source light 111 and (ii) the second light source light 121. The first light source light 111 has a first beam angle α1. The second light source light 121 has a second beam angle α2. The first beam angle α1 < the second beam angle α2. The beam angles are indicated with references α1 and α2. The beam width, e.g. the beam diameter in the case of an essentially circular cross-section, is indicated with references W1 and W2. Note that the beam angle of the beam of radiation may in embodiments especially be defined by the radiation component having the broadest beam angle (here α₂).

In embodiments, the first beam angle α1 may be selected from the range of at maximum 20°, like at maximum 15°. Additionally or alternatively, the second beam angle α2 may be selected from the range of at least 10°, like at least 15°. In specific embodiments, α2-α1≥5°. The radiation generating system 1000 is configured to provide the beam of radiation 1001 in an operational mode comprising both the first light source light 111 and the second light source light 121.

The radiation generating system 1000 may have an optical axis O. Each of the one or more first light sources 110 may have a first optical axis O1 and each of the one or more second light sources 120 may have a second optical axis O2. In embodiments, the one or more first light sources 110 may be configured closer to the optical axis O than the one or more second light sources 120. Thus, in embodiments, the distance d1 between the optical axis O and the one or more first optical axes O1 may be smaller than the distance d2 between the optical axis O and the one or more second optical axes O2.

In embodiments in an operational mode of the radiation generating system 1000 a beam of radiation 1001 is provided, with the beam of radiation 1001 comprising one or more of (i) the first beam 2111 comprising first light source light 111 having the first beam angle α1 defined by full width half maxima and (ii) the second beam 2121 comprising second light source light 121 having s the second beam angle α2 defined by full width half maxima. The first beam 2111 and the second beam 2121 at least partly overlap. Further, as indicated above, α1<α2. The first beam may have a first optical axis and the second beam may have a second optical axis. Especially, the optical axis of the first beam and the optical axis of the second beam may have a mutual angle αm, wherein 0°≤αm<α2-α1. Especially, in embodiments, the mutual angle may be essentially zero degrees. Especially, the first light source light and the second light source light are provided in essentially the same direction. Hence, the first beam 2111 and the second beam 2121 at least partly overlap. Even more especially, one of the first beam and the second beam may essentially overlap with the other of the first beam and the second beam. Especially, the first beam may essentially overlap with the broader second beam; the second beam may partly overlap with the more narrow first beam. Yet further, in embodiments the optical axis of the first beam and the second beam may essentially coincide. Hence, the first light and the second light may propagate in the same direction and at least partly overlap. In Fig. 1a, the optical axes of the first beam 2111 and the second beam 2121 essentially coincide with the optical axis O of the beam of radiation 1001.

The radiation generating system 1000 may in embodiments further comprise a beam shaping element 400, wherein the beam shaping element 400 may comprise one or more of a collimator, a reflector, a lens, and a lens array.

Fig. 1a schematically depicts an embodiment of the radiation generating system 1000 comprising one or more first light sources 110 and one or more second light sources 120. Especially, the one or more first light sources 110 and the one or more second light sources 120 are solid state light sources. Further, the one or more first light sources 110 may be configured to generate in an operational mode first light source light 111 having a first centroid wavelength λc1 defined within a wavelength range of at maximum 380 nm. Yet further, the one or more second light sources 120 may be configured to generate in an operational mode second light source light 121 having a second centroid wavelength λc2 defined within the wavelength range of at maximum 420 nm. Especially, λc2>λc1. Further, the radiation generating system 1000 may be configured to provide a beam of radiation 1001 comprising one or more of (i) the first light source light 111 and (ii) the second light source light 121. The first light source light 111 may have a first beam angle α1 and the second light source light 121 may have a second beam angle α2, and wherein α1<α2. The respective beam angles are defined by the respective full width half maxima.

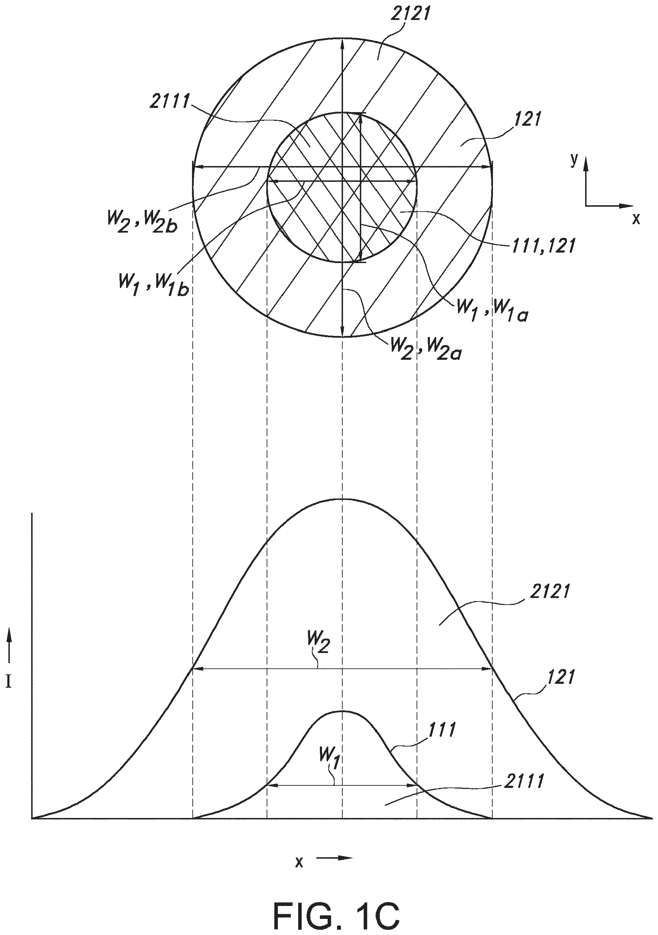

Referring to Fig. 1a, the 2111 and 2121 are at least partly overlapping. The former may be fully overlapped by the latter. Further, the schematically depicted embodiment shows symmetrical beams. Hence, the full width half maximum at one side of the optical axis O and the full width half maximum at the other side of the optical axis O define the beam angle (see also Fig. 1c).

Reference 5 indicates a surface on which the beam may be provided. On such surface, e.g. a spot may be seen see Fig. 1c.

Fig. 1b schematically depicts the emission peaks of the first light source light 111 and the second light source light 121. The one or more first light sources 110 may in embodiments be configured to generate in an operational mode first light source light 111 having a first centroid wavelength λc1 defined within a wavelength range of at maximum 420 nm, even more especially at maximum 380 nm. Additionally or alternatively, the one or more second light sources 120 may be configured to generate in an operational mode second light source light 121 having a second centroid wavelength λc2 defined within the wavelength range of at maximum 420 nm. In specific embodiments λc2>λc1.

The first centroid wavelength λc1 may in embodiments be selected within the wavelength range of 100-280 nm, especially in the range of 230-280 nm. Additionally or alternatively, the first centroid wavelength λc1 may in embodiments be selected within the wavelength range of 190-280 nm, especially within the wavelength range of 190-230 nm. However, other ranges may also be possible see below.

The second centroid wavelength λc2 may in embodiments be selected within the wavelength range of 280-420 nm. Additionally or alternatively, the second centroid wavelength λc2 may in embodiments be selected within the wavelength range of 230-280 nm. In specific embodiments, λc2- λc1≥30 nm.

References W1'and W2' indicate the spectral full width half maxima in this schematically (wavelength dependent) spectral power distribution.

Fig. 1c schematically depicts an embodiment of an operational mode of the disinfection device 1200, wherein both the first light source light 111 and the second light source light 121 have circular shaped cross-sections. For instance, this may be schematically depict a spot on a surface see also Fig. 1a.

The first light source light 111 may have a first full width half maximum W1. The second light source light 121 may have a second full width half maximum W2. In embodiments W1<W2. In alternative embodiments of an operational mode of the disinfection device 1200, the first light source light 111 and the second light source light 121 may have differently shaped cross-sections not depicted. Especially, in embodiments one of the first light source light 111 and the second light source light 121 may provide a circular cross-sectional beam shape and the other of the first light source light 111 and the second light source light 121 may provide a non-circular cross-sectional beam shape.

Here, circular cross-sections are depicted. In embodiments, the full width half maxima may be indicated along to orthogonal axis. In such embodiments, the full width half maxima for the first light source light may be indicated with references W1a and W1b, and the full width half maxima for the second light source light may be indicated with references W2a and W2b, respectively. For instance, W1a and W2a may be chosen parallel to each other (and thus W2a and W2b may also be chosen parallel to each other). As largest axis may be an axis chosen of the broadest part of the broadest beam.

Referring to Fig. 1c, the beams 2111 and 2121 are at least partly overlapping. The former may be fully overlapped by the latter. Further, the schematically depicted embodiment shows symmetrical beams. Hence, the full width half maximum at one side of the beam and the full width half maximum at another side of the beam may define the beam angle (see also Fig. 1c). As W1 indicates the full width half maximum, beyond W1 there is also intensity of first light; however, that intensity is lower than 50% of the maximum of beam 2111. Likewise, W2 indicates the full width half maximum, beyond W2 there is also intensity of first light; however, that intensity is lower than 50% of the maximum of beam 2121.

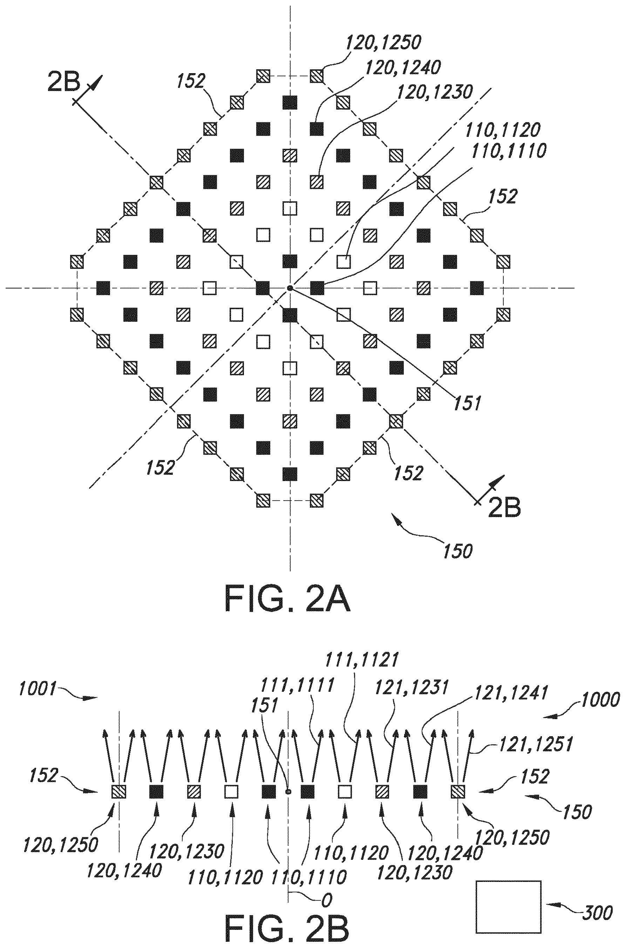

Fig. 2a schematically depicts an embodiment of the radiation generating system 1000 comprising a plurality of second light sources 120. The one or more first light sources 110 and the plurality of second light sources 120 may be configured in an array 150. The array 150 may have an array center 151, wherein the one or more first light sources 110 have a first average distance dₐ₁ to array center 151, and wherein the plurality of second light sources 120 have a second average distance dₐ₂ to array center 151. In embodiments, dₐ₁<dₐ₂. In embodiments, the array 150 may comprise an array edge 152, wherein a majority of the light sources 110,120 defining the array edge 152 may be selected from the group of violet light sources 1250.

In embodiments, the radiation generating system 1000 may comprise n11 Far UV-C light sources 1110 configured to generate far UV-C light 1111 having a centroid wavelength within the wavelength range of at maximum 230 nm. Additionally or alternatively, the radiation generating system 1000 may in embodiments comprise n12 near UV-C light sources 1120 configured to generate near UV-C light 1121 having a centroid wavelength within the wavelength range of 230-280 nm. Additionally or alternatively, the radiation generating system 1000 may in embodiments comprise n23 UV-B light sources 1230 configured to generate UV-B light 1231 having a centroid wavelength selected from the wavelength range of 280-315 nm. Additionally or alternatively, the radiation generating system 1000 may in embodiments comprise n24 UV-A light sources 1240 configured to generate UV-A light 1241 having a centroid wavelength selected from the wavelength range of 315-380 nm. Additionally or alternatively, the radiation generating system 1000 may in embodiments comprise n25 violet light sources 1250 configured to generate violet light 1251 having a centroid wavelength selected from the wavelength range of 380-420 nm. In specific embodiments n11≥0; n12≥0; n23≥0; n24≥0; n25≥0; n11+n12≥1 and wherein n23+n24+n25≥2. In specific embodiments, n11<n12. Additionally or alternatively, n25≥1.

Fig. 2b schematically depicts a cross-section of the array 150 depicted in Fig. 2a comprising the array center 151 and array edge 152. Further, schematically a control system 300 is depicted.

In embodiments, the control system 300 may be configured to individually control two or more of the (a) n11 Far UV-C light sources 1110, (b) n12 near UV-C light sources 1120, (c) n23 UV-B light sources 1230, (d) n24 UV-A light sources 1240, and (e) n25 violet light sources 1250.

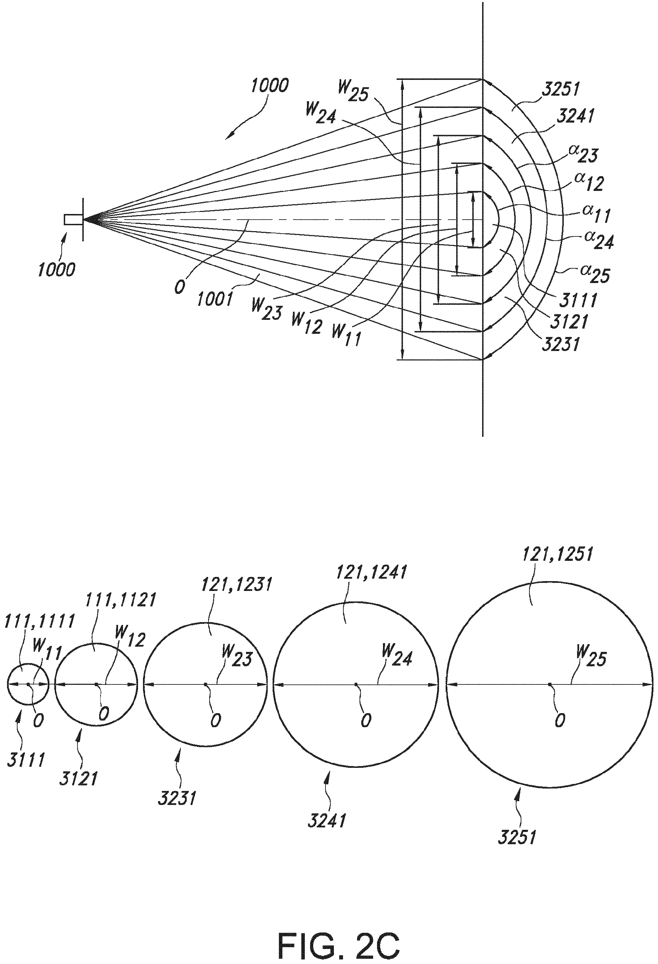

Figs. 2a and 2b, and also Fig. 2c, schematically depict embodiments of the radiation generating system 1000, comprising one or more of, especially two or more of (a) n11 Far UV-C light sources 1110 configured to generate far UV-C light 1111 having a centroid wavelength within the wavelength range of at maximum 230 nm, (b) n12 near UV-C light sources 1120 configured to generate near UV-C light 1121 having a centroid wavelength within the wavelength range of 230-280 nm, (c) n23 UV-B light sources 1230 configured to generate UV-B light 1231 having a centroid wavelength selected from the wavelength range of 280-315 nm, (d) n24 UV-A light sources 1240 configured to generate UV-A light 1241 having a centroid wavelength selected from the wavelength range of 315-380 nm, and (e) n25 violet light sources 1250 configured to generate violet light 1251 having a centroid wavelength selected from the wavelength range of 380-420 nm. Especially, n11≥0; n12≥0; n23≥0; n24≥0; n25≥0. In embodiments n11+n12≥1 and n23+n24+n25≥1, such as at least 2.

Fig. 2c, schematically depicts an embodiment of the radiation generating system 1000, such as described above, configured to generate (a) far UV-C light 1111 having a beam angle α11, (b) near UV-C light 1121 having a beam angle α12, (c) UV-B light 1231 having a beam angle α23, (d) UV-A light 1241 having a beam angle α24, and (e) violet light 1251 having a beam angle α25.

The relation first light sources 110, second light sources 120, and Far UV-C light sources 1110, UV-C light sources 1120, UV-B light sources 1230, UV-A light sources 1240, and violet light sources 1250, including a non-limiting number of embodiments (emb):

| | **first light sources 110** | **second light sources 120** | **Emb1** | **Emb2** | **Emb3** | **Emb4** | **Emb5** |
|---|---|---|---|---|---|---|---|
| Far UV-C light sources 1110 | X | | | X | X | | X |
| Near UV-C light sources 1120 | X | | X | X | X | X | X |
| UV-B light sources 1230 | | X | X | At least one of these three | X | X | |
| UV-A light sources 1240 | | X | At least one of these two | | X | | |
| violet light sources 1250 | | X | | | X | X | X |

Hence, in embodiments the radiation generating system 1000 may be configured to provide the beam of radiation 1001 comprising one or more of: (i) UV-C light 1121 having a beam angle α12, (ii) UV-B light 1231 having a beam angle α23, and (iii) UV-A light 1241 having a beam angle α24 and/or violet light 1251 having a beam angle α25. In specific embodiments, α12<α23<α24. Additionally or alternatively, α12<α23<α25. In further embodiments, n12≥1; n23≥1; n24+n25≥1.

The radiation generating system 1000 may in embodiments be configured to provide the beam of radiation 1001 comprising one or more of: (i) UV-C light 1121 having a beam angle α12, (ii) far UV-C light 1111 having a beam angle α11, and (iii) one or more of UV-B light 1231 having a beam angle α23, UV-A light 1241 having a beam angle α24 and violet light 1251 having a beam angle α25. In specific embodiments, α12<α11<α23. Additionally or alternatively, α12<α11<α24. Additionally or alternatively, α12<α11<α25. In further embodiments n11≥1; n12≥1; n24+n23+n25≥1.

Referring to Fig. 2c, the beam angle of the beam of radiation 1001 may especially be defined by the radiation component having the broadest beam angle (i.e. here α₂₅).

Referring to Fig. 2c, in embodiments in an operational mode of the radiation generating system a beam of radiation 1001 may be provided comprising one or more of (i) a far UV-C light beam 3111 comprising far UV-C light 1111 having a far UV-C light beam angle α11 defined by full width half maxima, (ii) a near UV-C light beam 3121 comprising near UV-C light 1121 having a near UV-C light beam angle α12 defined by full width half maxima, (iii) a UV-B light beam 3231 comprising UV-B light 1231 having a UV-B light beam angle α23 defined by full width half maxima, (iv) a UV-A light beam 3241 comprising UV-A light 1241 having a UV-A light beam angle α24 defined by full width half maxima, and (v) a violet light beam 3251 comprising violet light 1251 having a violet light beam angle α25 defined by full width half maxima. Especially, any combination of two beams at least partly overlaps.

Each of the beams may have an optical axis. In embodiments, any combination of two beams may have a mutual angle αₘ, wherein 0^{o}≤αₘ<|αₓ₁-αₓ₂|, wherein αₓ₁ indicates the beam angle of one of the beams and αₓ₂ the beam angle of the other of the beams.

As schematically depicted, the far UV-C light 1111, near UV-C light 1121, UV-B light 1231, UV-A light 1241, and violet light 1251 are provided in essentially the same direction. Hence, any combination of two beams of far UV-C light 1111, near UV-C light 1121, UV-B light 1231, UV-A light 1241, and violet light 1251 may at least partly overlap. Even more especially, one of the beams of far UV-C light 1111, near UV-C light 1121, UV-B light 1231, UV-A light 1241, and violet light 1251 may essentially overlap with another one of the beams of far UV-C light 1111, near UV-C light 1121, UV-B light 1231, UV-A light 1241, and violet light 1251. Yet further, in embodiments the optical axes of two or more, especially three or more, even more especially four or more, yet even more especially of all beams of far UV-C light 1111, near UV-C light 1121, UV-B light 1231, UV-A light 1241, and violet light 1251 may essentially coincide. Hence, the far UV-C light 1111, near UV-C light 1121, UV-B light 1231, UV-A light 1241, and violet light 1251 may propagate in the same direction and at least partly overlap.

Fig. 2c shows at the bottom the respective beams as defined by their respective full width half maxima.

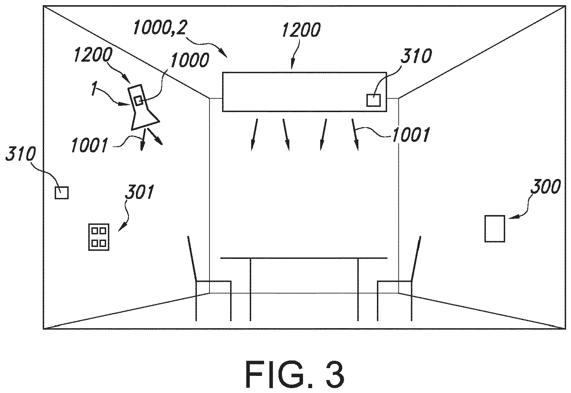

Fig. 3 schematically depicts an embodiment of a disinfection device 1200 comprising the radiation generating system 1000 providing system light 1001 as described above. The disinfection device 1200 may in embodiments have the shape of a spotlight, schematically depicted as lamp 1. Reference 301 indicates a user interface which may be functionally coupled with the control system 300 comprised by or functionally coupled to the radiation generating system 1000.

The control system 300 may in embodiments be configured to control one or more of a spectral power distribution of the system light 1001 and a spatial power distribution of the system light 1001. The radiation generating system 1000 may in embodiments further comprise a sensor 310, wherein the control system 300 may be configured to individually control the one or more first light sources 110 and the one or more second light sources 120 in dependence of a sensor signal of the sensor 310. In embodiments, the invention may further comprise a method for treating a gas or a surface in a space external from the radiation generating system 1000 or the disinfection device 1200. The method may especially comprise providing the radiation 1001 to the gas or the surface with the radiation generating system 1000 or the disinfection device 1200.

The term "plurality" refers to two or more.

The terms "substantially" or "essentially" herein, and similar terms, will be understood by the person skilled in the art. The terms "substantially" or "essentially" may also include embodiments with "entirely", "completely", "all", etc. Hence, in embodiments the adjective substantially or essentially may also be removed. Where applicable, the term "substantially" or the term "essentially" may also relate to 90% or higher, such as 95% or higher, especially 99% or higher, even more especially 99.5% or higher, including 100%.

The term "comprise" also includes embodiments wherein the term "comprises" means "consists of'.

The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices, apparatus, or systems may herein amongst others be described during operation. As will be clear to the person skilled in the art, the invention is not limited to methods of operation, or devices, apparatus, or systems in operation.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim.

Use of the verb "to comprise" and its conjugations does not exclude the presence of elements or steps other than those stated in a claim. Unless the context clearly requires otherwise, throughout the description and the claims, the words "comprise", "comprising", and the like are to be construed in an inclusive sense as opposed to an exclusive or exhaustive sense; that is to say, in the sense of "including, but not limited to".

The article "a" or "an" preceding an element does not exclude the presence of a plurality of such elements.

The invention may be implemented by means of hardware comprising several distinct elements, and by means of a suitably programmed computer. In a device claim, or an apparatus claim, or a system claim, enumerating several means, several of these means may be embodied by one and the same item of hardware. In yet a further aspect, the invention (thus) provides a software product, which, when running on a computer is capable of bringing about (one or more embodiments of) the method as described herein.

The invention also provides a control system that may control the device, apparatus, or system, or that may execute the herein described method or process. Yet further, the invention also provides a computer program product, when running on a computer which is functionally coupled to or comprised by the device, apparatus, or system, controls one or more controllable elements of such device, apparatus, or system.

The invention further applies to a device, apparatus, or system comprising one or more of the characterizing features described in the description and/or shown in the attached drawings. The invention further pertains to a method or process comprising one or more of the characterizing features described in the description and/or shown in the attached

## Claims

1. A radiation generating system (1000) comprising one or more first light sources (110) and one or more second light sources (120), wherein:
- the one or more first light sources (110) and the one or more second light sources (120) are solid state light sources;
- the one or more first light sources (110) are configured to generate in an operational mode first light source light (111) having a first centroid wavelength (λc1) defined within a wavelength range of at maximum 380 nm;
- the one or more second light sources (120) are configured to generate in an operational mode second light source light (121) having a second centroid wavelength (λc2) defined within the wavelength range of at maximum 420 nm; wherein the second centroid wavelength (λc2) > the first centroid wavelength (λc1);
- the radiation generating system (1000) is configured to provide a beam of radiation (1001) comprising one or more of (i) a first beam (2111) comprising first light source light (111) having a first beam angle (α1) defined by full width half maxima and (ii) a second beam (2121) comprising second light source light (121) having a second beam angle (α2) defined by full width half maxima; wherein the first beam (2111) and the second beam (2121) at least partly overlap, the radiation generating system being **characterized in that** the first beam angle (α1) < the second beam angle (α2).

2. The radiation generating system (1000) according to claim 1, wherein the first centroid wavelength (λc1) is selected within the wavelength range of 100-280 nm, wherein the second centroid wavelength (λc2) is selected within the wavelength range of 280-420 nm, and wherein the second centroid wavelength (λc2) - the first centroid wavelength (λc1) ≥ 30 nm; wherein the first beam angle (α1) is selected from the range of at maximum 15°, wherein the second beam angle (α2) is selected from the range of at least 10°, wherein the second beam angle (α2) - the first beam angle (α1) ≥ 5°; and wherein the radiation generating system (1000) is configured to provide the beam of radiation (1001) in an operational mode comprising both the first light source light (111) and the second light source light (121).

3. The radiation generating system (1000) according to any one of the preceding claims, wherein the first centroid wavelength (λc1) is selected within the wavelength range of 190-230 nm.

4. The radiation generating system (1000) according to any one of the preceding claims, having an optical axis (O), wherein the one or more first light sources (110) are configured closer to the optical axis (O) than the one or more second light sources (120).

5. The radiation generating system (1000) according to any one of the preceding claims, comprising a plurality of second light sources (120), wherein the one or more first light sources (110) and the plurality of second light sources (120) are configured in an array (150), wherein the array (150) has an array center (151), wherein the one or more first light sources (110) have a first average distance (dₐ₁) to array center (151), and wherein the plurality of second light sources (120) have a second average distance (dₐ₂) to array center (151), wherein the first average distance (dₐ₁) < the second average distance (dₐ₂).

6. The radiation generating system (1000) according to any one of the preceding claims, wherein the one or more first light sources (110) and the one or more second light sources (120) comprising two or more of (a) a number n11 of far UV-C light sources (1110) configured to generate far UV-C light (1111) having a centroid wavelength within the wavelength range of at maximum 230 nm, and (b) a number n12 of near UV-C light sources (1120) configured to generate near UV-C light (1121) having a centroid wavelength within the wavelength range of 230-280 nm, (c) a number n23 of UV-B light sources (1230) configured to generate UV-B light (1231) having a centroid wavelength selected from the wavelength range of 280-315 nm, (d) a number n24 of UV-A light sources (1240) configured to generate UV-A light (1241) having a centroid wavelength selected from the wavelength range of 315-380 nm, and (e) a number n25 of violet light sources (1250) configured to generate violet light (1251) having a centroid wavelength selected from the wavelength range of 380-420 nm, and wherein the number n11 ≥ 0; the number n12 ≥ 0; the number n23 ≥ 0; the number n24 ≥ 0; the number n25 ≥ 0; the number n11 + the number n12 ≥ 1 and wherein the number n23 + the number n24 + the number n25 ≥ 2.

7. The radiation generating system (1000) according to claim 6, wherein the radiation generating system (1000) is configured to provide the beam of radiation (1001) comprising: (i) near UV-C light (1121) having a beam angle (α12), (ii) UV-B light (1231) having a beam angle (α23), and (iii) UV-A light (1241) having a beam angle (α24) and/or violet light (1251) having a beam angle (α25), wherein the beam angle (α12) < the beam angle (α23) < the beam angle (α24) and/or the beam angle (α12) < the beam angle (α23) < the beam angle (α25).

8. The radiation generating system (1000) according to claim 6, wherein the radiation generating system (1000) is configured to provide the beam of radiation (1001) comprising: (i) near UV-C light (1121) having a beam angle (α12), (ii) far UV-C light (1111) having a beam angle (α11), and (iii) one or more of UV-B light (1231) having a beam angle (α23), UV-A light (1241) having a beam angle (α24) and violet light (1251) having a beam angle (α25), wherein the beam angle (α12) < the beam angle (α11) < the beam angle (α23) and/or the beam angle (α12) < the beam angle (α11) < the beam angle (α24) and/or the beam angle (α12) < the beam angle (α11) < the beam angle (α25).

9. The radiation generating system (1000) according to claim 5, and according to any one of the preceding claims 6-8, wherein the array (150) comprises an array edge (152), wherein a majority of the light sources (110,120) defining the array edge (152) are selected from the group of violet light sources (1250).

10. The radiation generating system (1000) according to any one of the preceding claims, further comprising a control system (300) configured to simultaneously control a spectral power distribution of the system light (1001) and a spatial power distribution of the system light (1001) from a first spectral power distribution and a first spatial power distribution to a second spectral power distribution different from said first spectral power distribution and a second spatial power distribution different from said first spatial power distribution, and wherein the system light (1001) comprises the first light source light (111) and the second light source light (121).

11. The radiation generating system (1000) according to claim 10, and according to any one of the preceding claims 6-8, wherein the control system (300) is configured to individually control two or more of the (a) the number n11 of far UV-C light sources (1110), (b) the number n12 of near UV-C light sources (1120), (c) the number n23 of UV-B light sources (1230), (d) the number n24 of UV-A light sources (1240), and (e) the number n25 of violet light sources (1250).

12. The radiation generating system (1000) according to claims 10-11, wherein the control system (300) configured to simultaneously control a spectral power distribution of the system light (1001) and a spatial power distribution of the system light (1001); wherein the control system (300) is configured to individually control three or more of the (a) the number n11 of far UV-C light sources (1110), (b) the number n12 of near UV-C light sources (1120), (c) the number n23 of UV-B light sources (1230), (d) the number n24 of UV-A light sources (1240), and (e) the number n25 of violet light sources (1250).

13. The radiation generating system (1000) according to any one of the preceding claims, comprising a beam shaping element (400), wherein the beam shaping element (400), the one or more first light sources (110) and the one or more second light sources (120) are configured such that the beam of first light source light (111) is more collimated than the beam of second light source light (121).

14. A disinfection device (1200) comprising the radiation generating system (1000) according to any one of the preceding claims, wherein the one or more first light sources (110) and the one or more second light sources (120) comprise one or more of LEDs and superluminescent diodes.

15. A method for treating a gas or a surface in a space external from the radiation generating system (1000) according to any one of the preceding claims 1-13 or the disinfection device (1200) according to claim 14, the method comprising providing the radiation (1001) to the gas or the surface with the radiation generating system (1000) or the disinfection device.

## Patentansprüche

1. Strahlungserzeugungssystem (1000), umfassend eine oder mehrere erste Lichtquellen (110) und eine oder mehrere zweite Lichtquellen (120), wobei:
- die eine oder mehreren ersten Lichtquellen (110) und die eine oder mehreren zweiten Lichtquellen (120) Festkörperlichtquellen sind;
- die eine oder mehreren ersten Lichtquellen (110) dazu konfiguriert sind, in einem Betriebsmodus erstes Lichtquellenlicht (111) mit einer ersten Schwerpunktwellenlänge (λc1) zu erzeugen, die innerhalb eines Wellenlängenbereichs von maximal 380 nm definiert ist;
- die eine oder mehreren zweiten Lichtquellen (120) dazu konfiguriert sind, in einem Betriebsmodus zweites Lichtquellenlicht (121) mit einer zweiten Schwerpunktwellenlänge (λc2) zu erzeugen, die innerhalb des Wellenlängenbereichs von maximal 420 nm definiert ist; wobei die zweite Schwerpunktwellenlänge (λc2) > der ersten Schwerpunktwellenlänge (λc1) ist;
- das Strahlungserzeugungssystem (1000) so konfiguriert ist, dass es einen Strahlungsstrahl (1001) bereitstellt, der eines oder mehrere der folgenden Elemente umfasst: (i) einen ersten Strahl (2111), der Licht einer ersten Lichtquelle (111) mit einem ersten Strahlwinkel (α1) umfasst, der durch die volle Breite der Halbmaxima definiert ist, und (ii) einen zweiten Strahl (2121), der Licht einer zweiten Lichtquelle (121) mit einem zweiten Strahlwinkel (α2) umfasst, der durch die volle Breite der Halbmaxima definiert ist; wobei sich der erste Strahl (2111) und der zweite Strahl (2121) zumindest teilweise überlappen, wobei das Strahlungserzeugungssystem **dadurch gekennzeichnet, dass** der erste Strahlwinkel (α1) < der zweite Strahlwinkel (α2).

2. Strahlungserzeugungssystem (1000) nach Anspruch 1, wobei die erste Schwerpunktwellenlänge (λc1) innerhalb des Wellenlängenbereichs von 100-280 nm ausgewählt ist, wobei die zweite Schwerpunktwellenlänge (λc2) innerhalb des Wellenlängenbereichs von 280-420 nm ausgewählt ist, und wobei die zweite Schwerpunktwellenlänge (λc2) - die erste Schwerpunktwellenlänge (λc1) ≥ 30 nm ist; wobei der erste Strahlwinkel (α1) aus dem Bereich von maximal 15° ausgewählt ist, wobei der zweite Strahlwinkel (α2) aus dem Bereich von mindestens 10° ausgewählt ist, wobei der zweite Strahlwinkel (α2) - der erste Strahlwinkel (α1) ≥ 5° ist; und wobei das Strahlungserzeugungssystem (1000) dazu konfiguriert ist, den Strahlungsstrahl (1001) in einem Betriebsmodus bereitzustellen, der sowohl das erste Lichtquellenlicht (111) als auch das zweite Lichtquellenlicht (121) umfasst.

3. Strahlungserzeugungssystem (1000) nach einem der vorstehenden Ansprüche, wobei die erste Schwerpunktwellenlänge (λc1) innerhalb des Wellenlängenbereichs von 190-230 nm ausgewählt ist.

4. Strahlungserzeugungssystem (1000) nach einem der vorstehenden Ansprüche eine optische Achse (O) aufweist, wobei die eine oder mehreren ersten Lichtquellen (110) näher an der optischen Achse (O) angeordnet sind als die eine oder mehreren zweiten Lichtquellen (120).

5. Strahlungserzeugungssystem (1000) nach einem der vorstehenden Ansprüche, umfassend eine Vielzahl von zweiten Lichtquellen (120), wobei die eine oder mehreren ersten Lichtquellen (110) und die Vielzahl von zweiten Lichtquellen (120) in einem Array (150) angeordnet sind, wobei das Array (150) ein Array-Zentrum (151) aufweist, wobei die eine oder mehreren ersten Lichtquellen (110) einen ersten durchschnittlichen Abstand (dₐ₁) zum Array-Zentrum (151) aufweisen, und wobei die Vielzahl von zweiten Lichtquellen (120) einen zweiten durchschnittlichen Abstand (dₐ₂) zum Array-Zentrum (151) aufweist, wobei der erste Durchschnittsabstand (dₐ₁) < der zweite Durchschnittsabstand (dₐ₂).

6. Strahlungserzeugungssystem (1000) nach einem der vorstehenden Ansprüche, wobei die eine oder mehreren ersten Lichtquellen (110) und die eine oder mehreren zweiten Lichtquellen (120) zwei oder mehr der folgenden Elemente umfassen: (a) eine Anzahl n11 von fernen UV-C-Lichtquellen (1110), die so konfiguriert sind, dass sie fernes UV-C-Licht (1111) mit einer Schwerpunktwellenlänge innerhalb des Wellenlängenbereichs von maximal 230 nm erzeugen, und (b) eine Anzahl n12 von nahen UV-C-Lichtquellen (1120), die so konfiguriert sind, dass sie nahes UV-C-Licht (1121) mit einer Schwerpunktwellenlänge innerhalb des Wellenlängenbereichs von 230-280 nm erzeugen, (c) eine Anzahl n23 von UV-B-Lichtquellen (1230), die so konfiguriert sind, dass sie UV-B-Licht (1231) mit einer Schwerpunktwellenlänge erzeugen, die aus dem Wellenlängenbereich von 280-315 nm ausgewählt ist, (d) eine Anzahl n24 von UV-A-Lichtquellen (1240), (1241) mit einer Schwerpunktwellenlänge, die aus dem Wellenlängenbereich von 315-380 nm ausgewählt ist, und (e) eine Anzahl n25 von violetten Lichtquellen (1250), die dazu konfiguriert sind, violettes Licht (1251) mit einer Schwerpunktwellenlänge zu erzeugen, die aus dem Wellenlängenbereich von 380-420 nm ausgewählt ist, und wobei die Anzahl n11 ≥ 0; die Anzahl n12 ≥ 0; die Anzahl n23 ≥ 0; die Anzahl n24 ≥ 0; die Anzahl n25 ≥ 0; die Anzahl n11 + die Anzahl n12 ≥ 1 und wobei die Anzahl n23 + die Anzahl n24 + die Anzahl n25 ≥ 2 ist.

7. Strahlungserzeugungssystem (1000) nach Anspruch 6, wobei das Strahlungserzeugungssystem (1000) so konfiguriert ist, dass es den Strahlungsstrahl (1001) bereitstellt, umfassend: (i) nahes UV-C-Licht (1121) mit einem Strahlwinkel (α12), (ii) UV-B-Licht (1231) mit einem Strahlwinkel (α23), und (iii) UV-A-Licht (1241) mit einem Strahlwinkel (α24) und/oder violettes Licht (1251) mit einem Strahlwinkel (α25), wobei der Strahlwinkel (α12) < der Strahlwinkel (α23) < der Strahlwinkel (α24) und/oder der Strahlwinkel (α12) < der Strahlwinkel (α23) < der Strahlwinkel (α25).

8. Strahlungserzeugungssystem (1000) nach Anspruch 6, wobei das Strahlungserzeugungssystem (1000) dazu konfiguriert ist, den Strahlungsstrahl (1001) bereitzustellen, umfassend: (i) nahes UV-C-Licht (1121) mit einem Strahlwinkel (α12), (ii) fernes UV-C-Licht (1111) mit einem Strahlwinkel (α11), und (iii) eines oder mehrere von UV-B-Licht (1231) mit einem Strahlwinkel (α23), UV-A-Licht (1241) mit einem Strahlwinkel (α24) und violettem Licht (1251) mit einem Strahlwinkel (α25), wobei der Strahlwinkel (α12) < der Strahlwinkel (α11) < der Strahlwinkel (α23) und/oder der Strahlwinkel (α12) < der Strahlwinkel (α11) < der Strahlwinkel (α24) und/oder der Strahlwinkel (α12) < der Strahlwinkel (α11) < der Strahlwinkel (α25).

9. Strahlungserzeugungssystem (1000) nach Anspruch 5 und nach einem der vorstehenden Ansprüche 6 bis 8, wobei das Array (150) eine Array-Kante (152) umfasst, wobei eine Mehrheit der Lichtquellen (110, 120), die die Array-Kante (152) definieren, aus der Gruppe der violetten Lichtquellen (1250) ausgewählt sind.

10. Strahlungserzeugungssystem (1000) nach einem der vorstehenden Ansprüche, das ferner ein Steuersystem (300) umfasst, das dazu konfiguriert ist, gleichzeitig eine spektrale Leistungsverteilung des Systemlichts (1001) und eine räumliche Leistungsverteilung des Systemlichts (1001) von einer ersten spektralen Leistungsverteilung und einer ersten räumlichen Leistungsverteilung auf eine zweite spektrale Leistungsverteilung, die sich von der ersten spektralen Leistungsverteilung unterscheidet, und eine zweite räumliche Leistungsverteilung, die sich von der ersten räumlichen Leistungsverteilung unterscheidet, zu steuern, und wobei das Systemlicht (1001) das erste Lichtquellenlicht (111) und das zweite Lichtquellenlicht (121) umfasst.

11. Strahlungserzeugungssystem (1000) nach Anspruch 10 und nach einem der vorstehenden Ansprüche 6 bis 8, wobei das Steuersystem (300) so konfiguriert ist, dass es zwei oder mehr der (a) Anzahl n11 der fernen UV-C-Lichtquellen (1110), (b) der Anzahl n12 der nahen UV-C-Lichtquellen (1120), (c) der Anzahl n23 der UV-B-Lichtquellen (1230), (d) die Nummer n24 der UV-A-Lichtquellen (1240) und (e) die Nummer n25 der violetten Lichtquellen (1250) einzeln steuert.

12. Strahlungserzeugungssystem (1000) nach den Ansprüchen 10 bis 11, wobei das Steuersystem (300) dazu konfiguriert ist, gleichzeitig eine spektrale Leistungsverteilung des Systemlichts (1001) und eine räumliche Leistungsverteilung des Systemlichts (1001) zu steuern; wobei das Steuersystem (300) dazu konfiguriert ist, drei oder mehr der folgenden Elemente einzeln zu steuern: (a) die Anzahl n11 von fernen UV-C-Lichtquellen (1110), (b) die Anzahl n12 von nahen UV-C-Lichtquellen (1120), (c) die Anzahl n23 von UV-B-Lichtquellen (1230), (d) die Anzahl n24 von UV-A-Lichtquellen (1240) und (e) die Anzahl n25 von violetten Lichtquellen (1250).

13. Strahlungserzeugungssystem (1000) nach einem der vorstehenden Ansprüche, umfassend ein Strahlformungselement (400), wobei das Strahlformungselement (400), die eine oder mehreren ersten Lichtquellen (110) und die eine oder mehreren zweiten Lichtquellen (120) so konfiguriert sind, dass der Strahl des Lichts der ersten Lichtquelle (111) stärker kollimiert ist als der Strahl des Lichts der zweiten Lichtquelle (121).

14. Desinfektionsvorrichtung (1200), umfassend das Strahlungserzeugungssystem (1000) nach einem der vorstehenden Ansprüche, wobei die eine oder mehreren ersten Lichtquellen (110) und die eine oder mehrere zweiten Lichtquellen (120) eine oder mehrere LEDs und Superlumineszenzdioden umfassen.

15. Verfahren zum Behandeln eines Gases oder einer Oberfläche in einem Raum außerhalb des Strahlungserzeugungssystems (1000) nach einem der vorstehenden Ansprüche 1 bis 13 oder der Desinfektionsvorrichtung (1200) nach Anspruch 14, wobei das Verfahren das Bereitstellen der Strahlung (1001) für das Gas oder die Oberfläche mit dem Strahlungserzeugungssystem (1000) oder der Desinfektionsvorrichtung umfasst.

## Revendications

1. Système de génération de rayonnement (1000) comprenant une ou plusieurs premières sources de lumière (110) et une ou plusieurs secondes sources de lumière (120), dans lequel :
- les une ou plusieurs premières sources de lumière (110) et les une ou plusieurs secondes sources de lumière (120) sont des sources de lumière à semiconducteurs ;
- les une ou plusieurs premières sources de lumière (110) sont conçues pour générer, dans un mode opérationnel, une lumière (111) de première source de lumière ayant une première longueur d'onde centroïde (λc1) définie au sein d'une plage de longueurs d'onde d'au maximum 380 nm ;
- les une ou plusieurs secondes sources de lumière (120) sont conçues pour générer, dans un mode opérationnel, une lumière (121) de seconde source de lumière ayant une seconde longueur d'onde centroïde (λc2) définie au sein d'une plage de longueurs d'onde d'au maximum 420 nm ; dans lequel la seconde longueur d'onde centroïde (λc2) > la première longueur d'onde centroïde (λc1) ;
- le système de génération de rayonnement (1000) est conçu pour fournir un faisceau de rayonnement (1001) comprenant un ou plusieurs parmi (i) un premier faisceau (2111) comprenant une première source de lumière (111) ayant un premier angle de faisceau (α1) défini par une largeur totale à mi-hauteur et (ii) un second faisceau (2121) comprenant une seconde source de lumière (121) ayant un second angle de faisceau (α2) défini par une largeur totale à mi-hauteur ; dans lequel le premier faisceau (2111) et le second faisceau (2121) se chevauchent au moins partiellement, le système de génération de rayonnement étant **caractérisé en ce que** le premier angle de faisceau (α1) < le second angle de faisceau (α2).

2. Système de génération de rayonnement (1000) selon la revendication 1, dans lequel la première longueur d'onde centroïde (λc1) est sélectionnée au sein de la plage de longueurs d'onde de 100 à 280 nm, dans lequel la seconde longueur d'onde centroïde (λc2) est sélectionnée au sein de la plage de longueurs d'onde de 280 à 420 nm, et dans lequel la seconde longueur d'onde centroïde (λc2) - la première longueur d'onde centroïde (λc1) ≥ 30 nm ; dans lequel le premier angle de faisceau (α1) est choisi dans la plage d'au maximum 15 °, dans lequel le second angle de faisceau (α2) est choisi dans la plage d'au moins 10 °, dans lequel le second angle de faisceau (α2) - le premier angle de faisceau (α1) ≥ 5 ° ; et dans lequel le système de génération de rayonnement (1000) est conçu pour fournir le faisceau de rayonnement (1001) dans un mode opérationnel comprenant à la fois la lumière (111) de première source de lumière et la lumière (121) de seconde source de lumière.

3. Système de génération de rayonnement (1000) selon l'une quelconque des revendications précédentes, dans lequel la première longueur d'onde centroïde (λc1) est sélectionnée au sein de la plage de longueurs d'onde de 190 à 230 nm.

4. Système de génération de rayonnement (1000) selon l'une quelconque des revendications précédentes, ayant un axe optique (O), dans lequel les une ou plusieurs premières sources de lumière (110) sont conçues plus près de l'axe optique (O) que les une ou plusieurs secondes sources de lumière (120).

5. Système de génération de rayonnement (1000) selon l'une quelconque des revendications précédentes, comprenant une pluralité de secondes sources de lumière (120), dans lequel les une ou plusieurs premières sources de lumière (110) et la pluralité de secondes sources de lumière (120) sont conçues dans un réseau (150), dans lequel le réseau (150) a un centre de réseau (151), dans lequel les une ou plusieurs premières sources de lumière (110) ont une première distance moyenne (dₐ₁) par rapport au centre de réseau (151), et dans lequel la pluralité de secondes sources de lumière (120) ont une seconde distance moyenne (dₐ₂) par rapport au centre de réseau (151), dans lequel la première distance moyenne (dₐ₁) < la seconde distance moyenne (dₐ₂).

6. Système de génération de rayonnement (1000) selon l'une quelconque des revendications précédentes, dans lequel les une ou plusieurs premières sources de lumière (110) et les une ou plusieurs secondes sources de lumière (120) comprennent deux ou plus parmi (a) un nombre n11 de sources de lumière UV-C lointaine (1110) conçues pour générer une lumière UV-C lointaine (1111) ayant une longueur d'onde centroïde au sein de la plage de longueurs d'onde d'au maximum 230 nm, et (b) un nombre n12 de sources de lumière UV-C proche (1120) conçues pour générer une lumière UV-C proche (1121) ayant une longueur d'onde centroïde au sein de la plage de longueurs d'onde de 230 à 280 nm, (c) un nombre n23 de sources de lumière UV-B (1230) conçues pour générer de la lumière UV-B (1231) ayant une longueur d'onde centroïde sélectionnée dans la plage de longueurs d'onde de 280 à 315 nm, (d) un nombre n24 de sources de lumière UV-A (1240) conçues pour générer de la lumière UV-A (1241) ayant une longueur d'onde centroïde sélectionnée dans la plage de longueurs d'onde de 315 à 380 nm, et (e) un nombre n25 de sources de lumière violette (1250) conçues pour générer de la lumière violette (1251) ayant une longueur d'onde centroïde sélectionnée dans la plage de longueurs d'onde de 380 à 420 nm, et dans lequel le nombre n11 ≥ 0 ; le nombre n12 ≥ 0 ; le nombre n23 ≥ 0 ; le nombre n24 ≥ 0 ; le nombre n25 ≥ 0 ; le nombre n11 + le nombre n12 ≥ 1 et dans lequel le nombre n23 + le nombre n24 + le nombre n25 ≥ 2.

7. Système de génération de rayonnement (1000) selon la revendication 6, dans lequel le système de génération de rayonnement (1000) est conçu pour fournir le faisceau de rayonnement (1001) comprenant : (i) de la lumière UV-C proche (1121) ayant un angle de faisceau (α12), (ii) de la lumière UV-B (1231) ayant un angle de faisceau (α23), et (iii) de la lumière UV-A (1241) ayant un angle de faisceau (α24) et/ou de la lumière violette (1251) ayant un angle de faisceau (α25), dans laquelle l'angle de faisceau (α12) < l'angle de faisceau (α23) < l'angle de faisceau (α24) et/ou l'angle de faisceau (α12) < l'angle de faisceau (α23) < l'angle de faisceau (α25).

8. Système de génération de rayonnement (1000) selon la revendication 6, dans lequel le système de génération de rayonnement (1000) est conçu pour fournir le faisceau de rayonnement (1001) comprenant : (i) de la lumière UV-C proche (1121) ayant un angle de faisceau (α12), (ii) de la lumière UV-C lointaine (1111) ayant un angle de faisceau (α11), et (iii) une ou plusieurs parmi de la lumière UV-B (1231) ayant un angle de faisceau (α23), de la lumière UV-A (1241) ayant un angle de faisceau (α24) et de la lumière violette (1251) ayant un angle de faisceau (α25), dans lequel l'angle de faisceau (α12) < l'angle de faisceau (α11) < l'angle de faisceau (α23) et/ou l'angle de faisceau (α12) < l'angle de faisceau (α11) < l'angle de faisceau (α24) et/ou l'angle de faisceau (α12) < l'angle de faisceau (α11) < l'angle de faisceau (α25).

9. Système de génération de rayonnement (1000) selon la revendication 5, et selon l'une des revendications précédentes 6 à 8, dans lequel le réseau (150) comprend un bord de réseau (152), dans lequel une majorité des sources de lumière (110, 120) définissant le bord de réseau (152) sont choisies dans le groupe des sources de lumière violette (1250).

10. Système de génération de rayonnement (1000) selon l'une quelconque des revendications précédentes, comprenant en outre un système de commande (300) conçu pour commander simultanément une distribution spectrale de puissance de la lumière de système (1001) et une distribution spatiale de puissance de la lumière de système (1001) d'une première distribution spectrale de puissance et d'une première distribution spatiale de puissance à une seconde distribution spectrale de puissance différente de ladite première distribution spectrale de puissance et une seconde distribution spatiale de puissance différente de ladite première distribution spatiale de puissance, et dans lequel la lumière de système (1001) comprend la lumière (111) de première source de lumière et la lumière (121) de seconde source de lumière.

11. Système de génération de rayonnement (1000) selon la revendication 10, et selon l'une quelconque des revendications précédentes 6 à 8, dans lequel le système de commande (300) est conçu pour commander individuellement deux ou plus parmi (a) le nombre n11 de sources de lumière UV-C lointaine (1110), (b) le nombre n12 de sources de lumière UV-C proche (1120), (c) le nombre n23 de sources de lumière UV-B (1230), (d) le nombre n24 de sources de lumière UV-A (1240), et (e) le nombre n25 de sources de lumière violette (1250).

12. Système de génération de rayonnement (1000) selon les revendications 10 à 11, dans lequel le système de commande (300) est conçu pour commander simultanément une distribution spectrale de puissance de la lumière de système (1001) et une distribution spatiale de puissance de la lumière de système (1001) ; dans lequel le système de commande (300) est conçu pour commander individuellement trois ou plus parmi (a) le nombre n11 de sources de lumière UV-C lointaine (1110), (b) le nombre n12 de sources de lumière UV-C proche (1120), (c) le nombre n23 de sources de lumière UV-B (1230), (d) le nombre n24 de sources de lumière UV-A (1240), et (e) le nombre n25 de sources de lumière violette (1250).

13. Système de génération de rayonnement (1000) selon l'une quelconque des revendications précédentes, comprenant un élément de mise en forme de faisceau (400), dans lequel l'élément de mise en forme de faisceau (400), les une ou plusieurs premières sources de lumière (110) et les une ou plusieurs secondes sources de lumière (120) sont conçus de telle sorte que le faisceau de la lumière (111) de première source de lumière est plus collimaté que le faisceau de la lumière (121) de seconde source de lumière.

14. Dispositif de désinfection (1200) comprenant le système de génération de rayonnement (1000) selon l'une quelconque des revendications précédentes, dans lequel les une ou plusieurs premières sources de lumière (110) et les une ou plusieurs secondes sources de lumière (120) comprennent une ou plusieurs parmi des DEL et des diodes superluminescentes.

15. Procédé de traitement d'un gaz ou d'une surface dans un espace extérieur au système de génération de rayonnement (1000) selon l'une quelconque des revendications précédentes 1 à 13 ou au dispositif de désinfection (1200) selon la revendication 14, le procédé comprenant la fourniture du rayonnement (1001) au gaz ou à la surface avec le système de génération de rayonnement (1000) ou le dispositif de désinfection.
